# EUROPEAN PATENT APPLICATION

(11) **EP 2 270 229 A1**
(43) Date of publication of application: **05.01.2011**
(21) Application number: 10172229.6
(22) Date of filing: 12.09.2007
(51) Int. Cl.: C12Q 1/68, C07H 21/02, C07H 21/04

(54) **Inhibitors of bone morphogenetic protein (BMP) signalling for therapeutical purposes**

(30) Priority: 12.09.2006 US 844038 P
(62) Divisional of application: 07838105.0
(71) Applicant: The General Hospital Corporation, Boston, MA 02114 (US)
(72) Inventor: Yu, Paul B., Boston, MA 02114 (US); Hong, Charles C., Nolensville, TN 37135 (US); Bloch, Kenneth D., Brookline, MA 02445 (US); Peterson, Randall T., Belmont, MA 02478 (US)
(74) Representative: Dörries, Hans Ulrich

(57) **Abstract**

The invention provides methods for identifying compounds that modulate cell signaling, as well as therapeutic methods that employ such compounds.

SEQUENCE LISTING

<110> THE GENERAL HOSPITAL CORPORATION

<120> METHODS FOR IDENTIFYING COMPOUNDS THAT MODULATE CELL SIGNALING AND
      METHODS EMPLOYING SUCH COMPOUNDS

<130> GHC13152PCTEP

<140> EP 07838105.0
<141> 2009-02-05

<150> PCT/US2007/019831
<151> 2007-09-12

<150> 60/844,038
<151> 2006-09-12

<160> 2

<170> PatentIn version 3.5

<210> 1
<211> 25
<212> DNA
<213> Homo sapiens

<400> 1
caccctggaa agttttacct ttgac                                           25


<210> 2
<211> 25
<212> DNA
<213> Homo sapiens

<400> 2
taaatgaaca gacttaactc ttcac                                           25

## Description

### Background of the Invention

This invention relates to methods for identifying compounds that modulate cell signaling, as well as methods employing such compounds.

Signaling involving the Transforming Growth Factor (TGF) superfamily of ligands is central to a wide range of cellular processes, including cell growth, differentiation, and apoptosis. TGF signaling involves binding of a TGF ligand to a type II receptor (a serine/threonine kinase), which recruits and phosphorylates a type I receptor. The type I receptor then phosphorylates a receptor-regulated SMAD (R-SMAD; e.g., SMAD1, SMAD2, SMAD3, SMAD5, SMAD8 or SMAD9), which binds to a co-SMAD, and the SMAD complex then enters the nucleus where it plays a role in transcriptional regulation. The TGF superfamily of ligands includes two major branches, characterized by TGF-β/activinlnodal and Bone Morphogenetic Proteins (BMPs).

Signals mediated by bone morphogenetic protein (BMP) ligands serve diverse roles throughout the life of vertebrates. During embryogenesis, the dorsoventral axis is established by BMP signaling gradients formed by the coordinated expression of ligands, receptors, co-receptors, and soluble antagonists (Massague et al., Nat. Rev. Mol. Cell. Biol. 1:169-178, 2000). Excess BMP signaling causes ventralization, an expansion of ventral at the expense of dorsal structures, while diminished BMP signaling causes dorsalization, an expansion of dorsal at the expense of ventral structures (Nguyen et al., Dev. Biol. 199:93-110, 1998; Furthauer et al., Dev. Biol. 214:181-196, 1999; Mintzer et al., Development 128:859-869,2001; Schmid et al., Development 127:957-967, 2000). BMPs are key regulators of gastrulation, mesoderm induction, organogenesis, and endochondral bone formation, and regulate the fates of multipotent cell populations (Zhao, Genesis 35:43-56, 2003). BMP signals also play critical roles in physiology and disease, and are implicated in primary pulmonary hypertension, hereditary hemorrhagic telangiectasia syndrome, fibrodysplasia ossificans progressiva, and juvenile polyposis syndrome (Waite et al., Nat. Rev. Genet. 4:763-773, 2003; Papanikolaou et al., Nat. Genet. 36:77-82, 2004; Shore et al., Nat. Genet. 38:525-527, 2006).

The BMP signaling family is a diverse subset of the transforming growth factor-β TGF-β) superfamily (Sebald et al., Biol. Chem. 385:697-710, 2004). Over twenty known BMP ligands are recognized by three distinct type II (BMPRII, ActRIIa, and ActRIIb) and at least three type I (ALK2, ALK3, and ALK6) receptors. Dimeric ligands facilitate assembly of receptor heteromers, allowing the constitutively-active type II receptor serine/threonine kinases to phosphorylate type I receptor serine/threonine kinases. Activated type I receptors phosphorylate BMP-responsive (BR-) SMAD effectors (SMADs 1, 5, and 8) to facilitate nuclear translocation in complex with SMAD4, a co-SMAD that also facilitates TGF signaling. In addition, BMP signals can activate intracellular effectors such as MAPK p38 in a SMAD-independent manner (Nohe et al., Cell Signal 16:291-299, 2004). Soluble BMP antagonists such as noggin, chordin, gremlin, and follistatin limit BMP signaling by ligand sequestration. Co-receptors such as members of the repulsive guidance molecule (RGM) family, including RGMa, DRAGON (RGMb), and hemojuvelin (HJV, RGMc), enhance the response to low levels of BMP ligands in the target tissues where they are expressed (Babitt et al., Nat. Genet. 38:531-539, 2006; Babitt et al., J. Biol. Chem. 280:29820-29827, 2005).

A role for BMP signals in regulating expression of hepcidin, a peptide hormone and central regulator of systemic iron balance, has been suggested (Pigeon et al., J. Biol. Chem. 276:7811-7819, 2001; Fraenkel et al., J. Clin. Invest. 115:1532-1541, 2005; Nicolas et al., Proc. Natl. Acad. Sci. U.S.A. 99:4596-4601, 2002; Nicolas et al., Nat. Genet. 34:97-101, 2003). Hepcidin binds and promotes degradation of ferroportin, the sole iron exporter in vertebrates. Loss of ferroportin activity prevents mobilization of iron to the bloodstream from intracellular stores in enterocytes, macrophages, and hepatocytes (Nemeth et al., Science 306:2090-2093, 2004). Hepatic hepcidin expression is responsive to states of iron deficiency or overload, inflammation, and hypoxia, but the mechanisms by which the liver senses these stimuli and regulates hepcidin expression remain unclear, especially because the promoter for the *HAMP* gene (which encodes hepcidin) does not appear to possess elements known to be modulated by iron levels. Mice conditionally deficient in SMAD4 fail to induce hepcidin following iron or interleukin-6 (IL-6) challenge (Nemeth et al., Science 306:2090-2093, 2004), suggesting that BMP and/or TGF-β pathways transduce signals mediated by iron or inflammation. In addition, mutations in *Hfe2* (which encodes the BMP co-receptor HJV) have been identified in juvenile hemochromatosis, a disease in which iron overload fails to trigger hepcidin expression (Papanikolaou et al., Nat. Genet. 36:77-82, 2004). This finding further suggests a link between BMP signaling and iron metabolism, but the role of BMPs in the modulation of hepcidin expression by iron or inflammation is still incompletely understood.

Aberrant TGF-β signaling has been implicated in numerous human diseases as well. For instance, TGF-β upregulation may underlie pathophysiologic changes in the aorta of Marfan's patients, and TGF-β is thought to be a key mediator of fibrosis seen in fibrotic diseases of the kidney, liver, and lung. Thus, the ability to specifically inhibit TGF-β signaling would provide approaches to treating these conditions and for determining their causes.

Given the tremendous structural diversity of the BMP and TGF-β superfamily at the level of ligands (>25 distinct ligands at present) and receptors (3 type I; 3 type II receptors that recognize BMPs), and the heterotetrameric manner of receptor binding, traditional approaches for inhibiting BMP signals via soluble receptor, endogenous inhibitors, or neutralizing antibodies are not practical or effective. Endogenous inhibitors such as noggin and follistatin have limited specificity for ligand subclasses. Single receptors have limited affinity for ligand, whereas ligand heterotetramers exhibit rather precise specificity for particular ligands. Neutralizing antibodies are specific for particular ligands or receptors and are also limited by the structural diversity of this signaling system.

Pharmacologic agents specifically antagonizing BMP or TGF-β signaling pathways could be used to manipulate these pathways in therapeutic or experimental applications. However, traditional biochemical approaches to identify small molecules with specificity for either TGF-β or BMP pathways have been hampered by the overall structural similarities of TGF-βBMP receptors. An exception is SB-431542, which is a selective inhibitor of TGF-β1 activin receptor-like kinases (ALKs), without having an effect on receptors that mediate BMP signaling. Approaches enabling the identification of compounds that are specific for BMP or TGF-β signaling pathways would be beneficial in the development of therapeutic agents for use in treating diseases and conditions associated with these pathways, such as those listed above.

### Summary of the Invention

The invention is based on (i) the development of a whole-animal (e.g., zebrafish) screen, (ii) the development of a cell-based ELISA screen, and (iii) the identification of a novel class of BMP inhibitors.

Accordingly, in a first aspect, the invention provides methods of identifying compounds that inhibit Bone Morphogenetic Protein (BMP) or Transforming Growth Factor-β (TGF- β) signaling. The methods can include contacting a zebrafish embryo with a candidate compound and observing the phenotype of the embryo. In such methods, detection of dorsalization of the embryo, relative to an untreated control, indicates the identification of a compound that inhibits BMP signaling, while detection of cyclopia or reduction in head size, relative to an untreated control, indicates the identification of a compound that inhibits TGF-β signaling.

In a second aspect, the invention provides methods of identifying compounds that modulate BMP or TGF-β signaling, involving the following steps: (i) contacting cells with a candidate compound; (ii) permeabilizing the plasma and nuclear membranes of the cells and precipitating proteins in the cells by methanol treatment (using, e.g., ice-cold methanol treatment and/or 10-15 minutes of methanol treatment); (iii) cross-linking precipitated proteins with glutaraldehyde (using, e.g., a solution of 0.25% to 2% glutaraldehyde and/or 10-15 minutes of treatment); (iv) quenching the glutaraldehyde with a bifunctional amine (e.g., lysine, ethylenediamine, and a phenylenediamine, which are used at a concentration of 25-200 mM, and at a pH of 8-10); and (v) determining the effect of the candidate compound on phosphorylation of SMAD1/5/8 or SMAD2 in compound treated cells (using, e.g., Enzyme Linked Immunosorbent Assay (ELISA) analysis), relative to untreated cells. In such methods, detection of increased phosphorylation of SMAD1/5/8 indicates the identification of a compound that activates BMP signaling, detection of decreased phosphorylation of SMAD1/5/8 indicates the identification of a compound that inhibits BMP signaling, detection of increased phosphorylation of SMAD2 indicates the identification of a compound that activates TGF-β signaling, and detection of decreased phosphorylation of SMAD2 indicates the identification of a compound that inhibits TGF-β signaling.

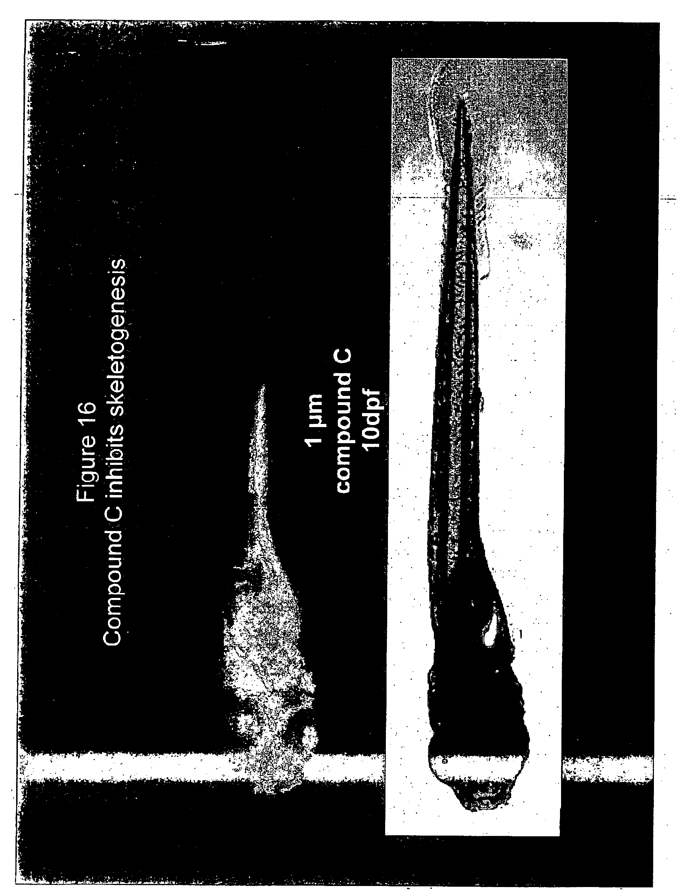

In a third aspect, the invention includes methods of treating or preventing diseases or conditions in subjects that would benefit by inhibition of BMP signaling, which involve administering to a subject one or more compounds that selectively inhibit BMP signaling, relative to TGF-β signaling. In one example, the compound is 6-[4-(2-piperidin-1-yl-ethoxy)-phenyl)]-3-pyridin-4-yl-pyrrazolo[1,5-a]-pyrimidine (referred to herein as "Compound C" and "dorsomorphin"), while in other examples the compound is selected from those shown in Figure 16. The diseases or conditions treated or prevented according to these methods can be, for example, selected from the group consisting of familial or sporadic primary pulmonary hypertension, cardiac valvular malformations, cancer (e.g., breast carcinoma, prostate carcinoma, renal cell carcinoma, bone metastasis, osteosarcoma, and multiple myeloma), anemia, vascular calcification, atherosclerosis, valve calcification, renal osteodystrophy, inflammatory disorders (e.g., ankylosing spondylitis), and Fibrodysplasia Ossificans Progressiva. The invention also includes use of BMP and TGF-β signaling modulators, as described herein, in the prevention and treatment of diseases and conditions such as those described herein.

In a fourth aspect, the invention includes methods of treating or preventing diseases or conditions in subjects that would benefit by inhibition of TGF-β signaling, which involve administering to a subject one or more compounds that selectively inhibit TGF-β signaling, relative to BMP signaling. The diseases or conditions treated or prevented according to these methods can be, for example, a fibrotic disease (of, e.g., the liver, kidneys, or lungs) or a valvular or aortic abnormality of Marfan's syndrome.

The invention also includes use of BMP and TGF-β signaling modulators, as described herein, in the prevention and treatment of diseases and conditions such as those described herein.

In a fifth aspect, the invention includes pharmaceutical compositions including compounds that modulate BMP and TGF-β signaling, as described herein. In one example, the compound is 6-[4-(2-piperidin-1-yl-ethoxy)-phenyl)]-3-pyridin-4-yl-pyrrazolo[1,5-a]-pyrimidine (Compound C/Dorsomorphin), which inhibits BMP.

In a sixth aspect, the invention provides methods of modifying the phenotype of a cell (e.g., an embryonic stem cell or an adult stem cell). These methods involve contacting the cell with a Bone Morphogenetic Protein (BMP) inhibitor (e.g., a BMP inhibitor identified using the methods described herein, such as, for example, 6-[4-(2-piperidin-1-yl-ethoxy)-phenyl)]-3-pyridin-4-yl-pyrrazolo[1,5-a]-pyrimidine (Compound C/Dorsomorphin). Under certain conditions, the inhibition of BMP signaling in embryonic stem cells can be used to promote differentiation, as BMP4 signaling acts to maintain pluripotency (Qi et al., Proc. Natl. Acad. Sci. U.S.A. 101:6027-6032, 2004). Yet under other conditions, inhibition of BMP signaling in embryonic stem cells can be used to help to maintain pluripotency, when BMP signaling acts to induce differentiation (Xu et al., Nat. Methods 2:185-190, 2005; Xu et al., Nat. Biotechnol. 20:1261-1264, 2002).

The invention provides several advantages. For example, the screening methods of the invention facilitate the identification of compounds that are specific for BMP or TGF-β signaling pathways. As discussed elsewhere herein, identifying compounds with specificity for BMP or TGF-β signaling has been challenging in the past, due to the complexities and high degree of similarities of these pathways. However, obtaining such specificity is important, as these pathways have opposing activities in some contexts.

In addition to facilitating the identification of compounds that are specific for BMP or TGF-β signaling, the screening methods of the invention also provide other advantages. For example, using the whole animal-based methods, which involve analysis of classic morphologies observed in treated embryos, one is assured early in the lead compound development stage that the degree of selectivity for BMP or TGF-β signaling pathways is sufficient to achieve specific effects in vivo. Further, as discussed in detail below, the cell culture-based methods provide advantages with respect to specificity, speed, and cost.

Small molecule modulators of BMP or TGF-β signaling, as can be identified according to the invention, provide advantages over recombinant endogenous BMP inhibitors, such as noggin and follistatin, as the latter would be very expensive to administer in vivo, requiring the intravenous dosing of gram quantities of protein, which may be hydrophobic or have stability problems in solution. In addition, recombinant endogenous BMP inhibitors may be restricted in their specificity to a small subset of the diversity of BMP ligands. Further, another limitation to the use of recombinant proteins as therapeutic agents is their potential for eliciting an immune response, which may limit their efficacy and/or precipitate illness in the recipient. An additional advantage of small molecule modulators is that they may be orally available, thus facilitating administration and treatment compliance. Use of Compound C/Dorsomorphin in therapeutic methods is advantageous, not only because of its specificity for BMP signaling, but it has been shown that mice can tolerate doses of 10 to 30 mg/kg each day without obvious toxicity (Kim et al., J. Biol. Chem. 279:19970-19976,2004).

Other features and advantages of the invention will be apparent from the following detailed description, the drawings, and the claims.

### Brief Description of the Drawings and the Tables

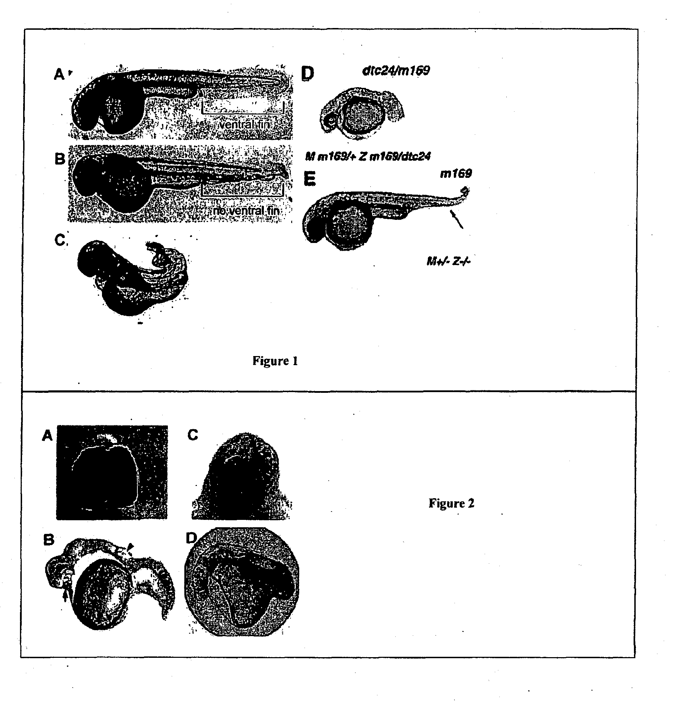

Figure 1 is a series of images showing the dorsalization of zebrafish embryos. (Panels D-E are reproduced and adapted from Kramer et al., Dev. Biol. 250:263-279, 2002.) A. Wild type embryo at 36 hours post fertilization (hpf) demonstrates normal head, tail, and ventral fin structures. B-C. Embryos treated with selective BMP inhibitor demonstrate decreased ventral fin development at 36 hpf, or in other instances severely attenuated tail development at 24 hpf, both consistent with dorsalization and specific inhibition of BMP signaling. D-E. Dorsalized embryos carrying different smad5 alleles. Embryos treated with selective BMP inhibitor closely mimic the range of dorsalization produced in by mutations in smad5 gene.
Figure 2 is a series of images showing the cyclopic phenotype. (Panels A-B are reproduced and adapted from Tian et al., Development 130:3331-3342, 2003; and Feldman et al., Nature 395:181-185, 1998, respectively.) A. Embryo carrying mutation in cyclops (a TGF-β-like) gene. B. Squint and cyclops double mutant embryo. Squint gene also encodes a member of the TGF-β gene family. C-D. Embryos treated with selective TGF-β inhibitor demonstrate cyclopia and great reduction in head size.

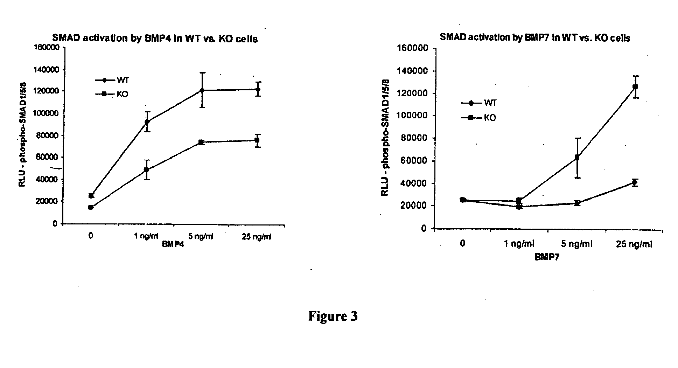

Figure 3 is two graphs showing sensitive detection by cellular ELISA of BMP-4-mediated SMAD activation in wild type (WT) and BMP type II receptor knockout (KO) cells (left panel). KO cells exhibit increased sensitivity to the effects of BMP7 (right panel), as previously shown (Yu et al., J. Biol. Chem. 280:24443-24450, 2005).

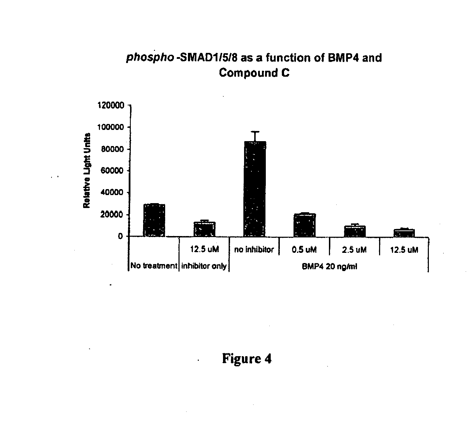

Figure 4 is a graph showing that activation of SMAD1/5/8 by BMP4 assayed by cellular ELISA is potently and preferentially inhibited by Compound C/Dorsomorphin. Compound C/Dorsomorphin potently inhibits the activation of the BMP signaling pathway with an IC₅₀ of less than 0.2 µM.

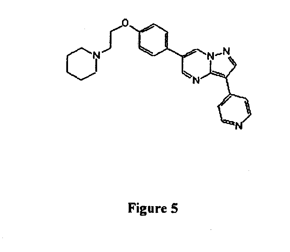

Figure 5 is a schematic illustration of the structure of Compound C/Dorsomorphin. This compound has been previously described as an inhibitor of AMP kinase activity with an IC₅₀ of approximately 15-30 µM for that pathway (Zhou et al., J. Clin. Invest. 108:1167-1174, 2001).

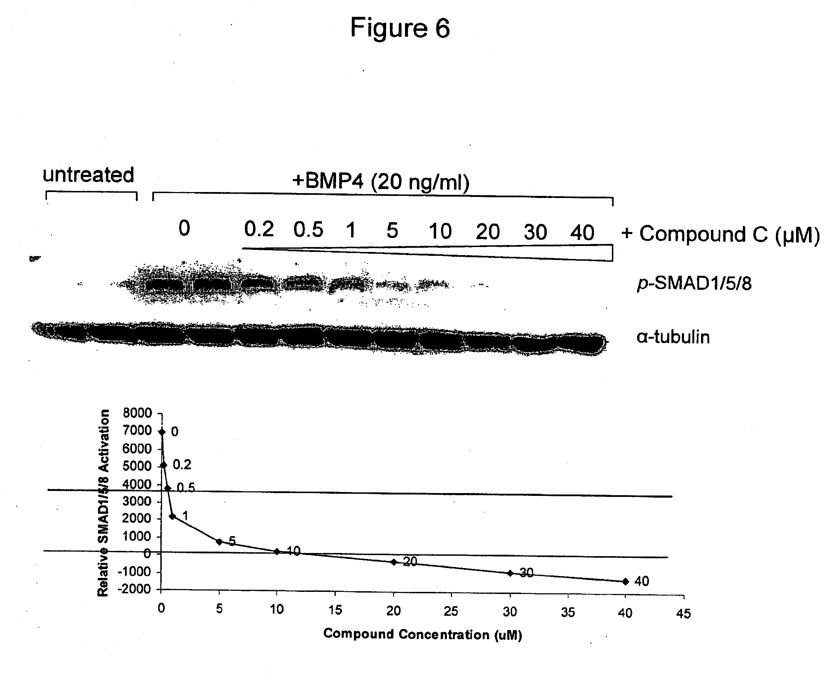

Figure 6 shows that activation of SMAD1/5/8 by BMP4 is preferentially inhibited by Compound C/Dorsomorphin.

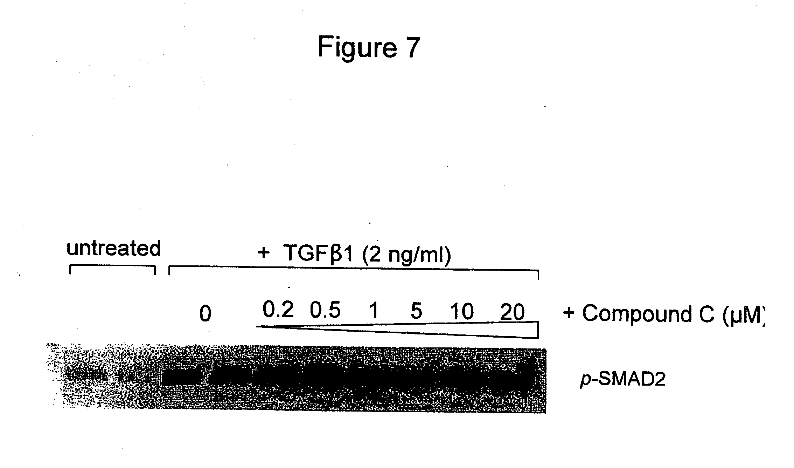

Figure 7 shows that Compound C/Dorsomorphin does not affect the TGF-β signaling pathway.

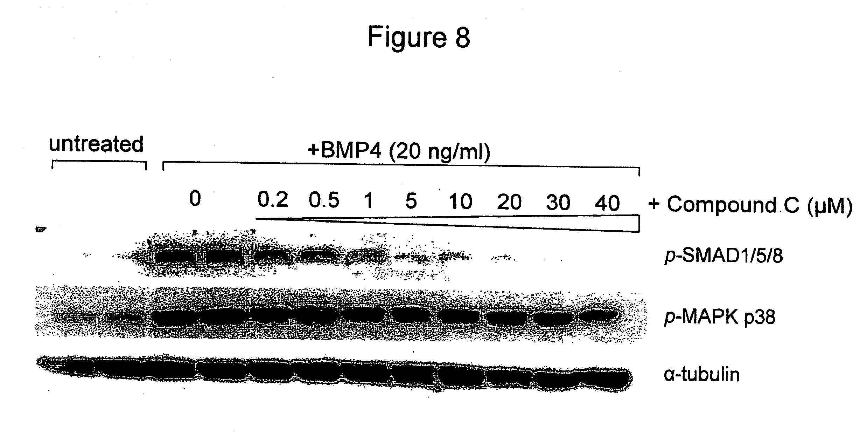

Figure 8 shows that Compound C/Dorsomorphin does not inhibit MAP kinase activation mediated by BMPs. BMP4 (20 ng/ml) induced phosphorylation of SMAD1/5/8 and p38 measured by Western blot in PASMCs, as previously shown (Yu et al., J. Biol. Chem. 280:24443-24450, 2005). Compound C/Dorsomorphin (0.2 µM to 40 µM) inhibited activation of SMADs 1/5/8 dose dependently without inhibiting p38 activation.

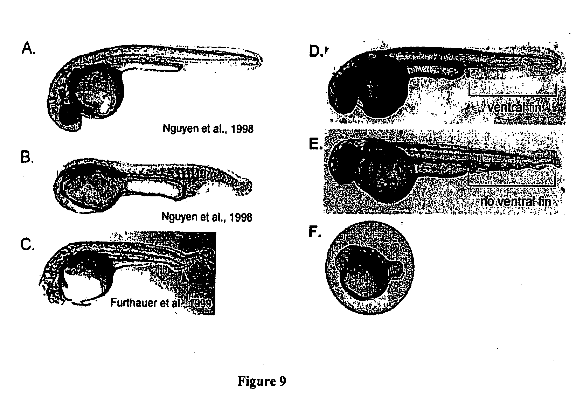

Figure 9 is a series of images showing that Compound C/Dorsomorphin induces dorsalization in developing zebrafish embryos. (Panels A-C are reproduced and adapted from Nguyen et al., Dev. Biol. 199:93-110, 1998, and Furthauer et al., Dev. Biol. 214:181-196, 1999) A. Wild type embryo at 36 hours post fertilization (hpf) demonstrates normal head, tail, and ventral fin structures. B. Overexpression of BMP2b demonstrates ventralization, with excess development of tail and tail fin structures, and diminished anterior development at 36 hpf. C. Overexpression of noggin, a selective inhibitor of BMP2 and BMP4, induces dorsalization, with preserved anterior structures but decreased tail and ventral fin development at 36 hpf. D. Normal wild type embryo at 36 hpf. E-F. Embryos treated with 5-20 µM Compound C/Dorsomorphin demonstrate decreased ventral fin development at 36 hpf, or in other instances severely attenuated tail development at 24 hpf, both consistent with dorsalization and specific inhibition of BMP signaling.

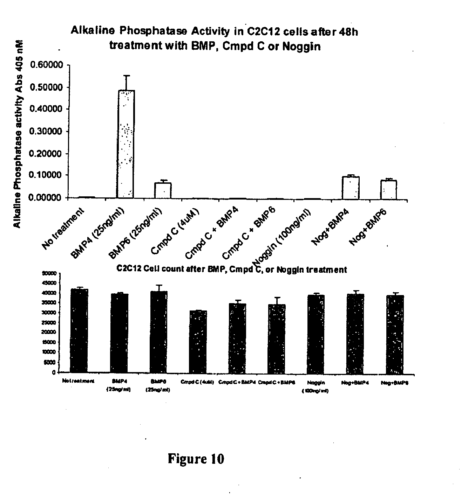

Figure 10 is two graphs showing that Compound C/Dorsomorphin inhibits BMP-mediated osteogenic differentiation in C2C12 cells without cellular toxicity.

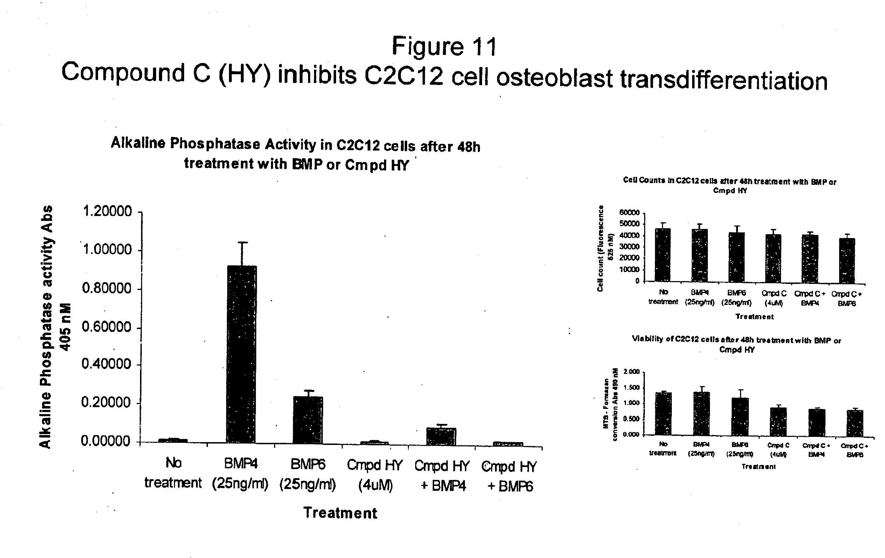

Figure 11 is a series of graphs showing that Compound C/Dorsomorphin inhibits C2C12 cell osteoblast transdifferentiation.

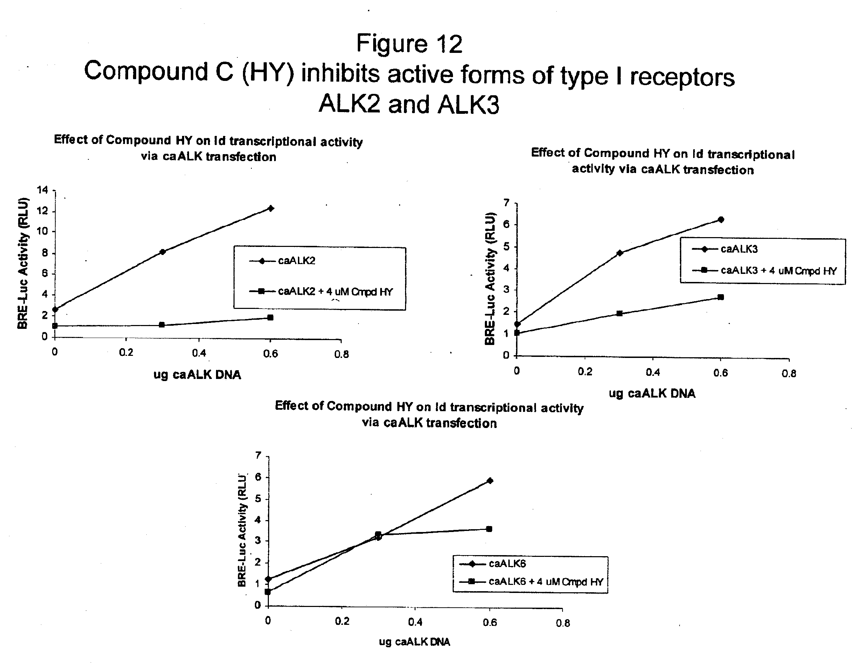

Figure 12 is a series of graphs showing that Compound C/Dorsomorphin inhibits active forms of type I receptors ALK2 and ALK3.

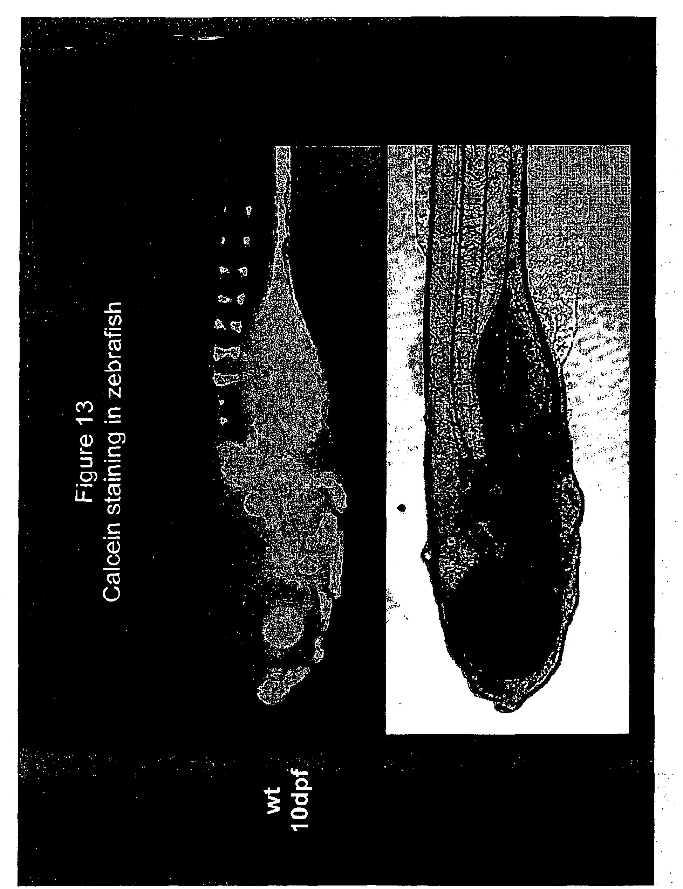

Figure 13 is an image showing calcein staining in zebrafish.

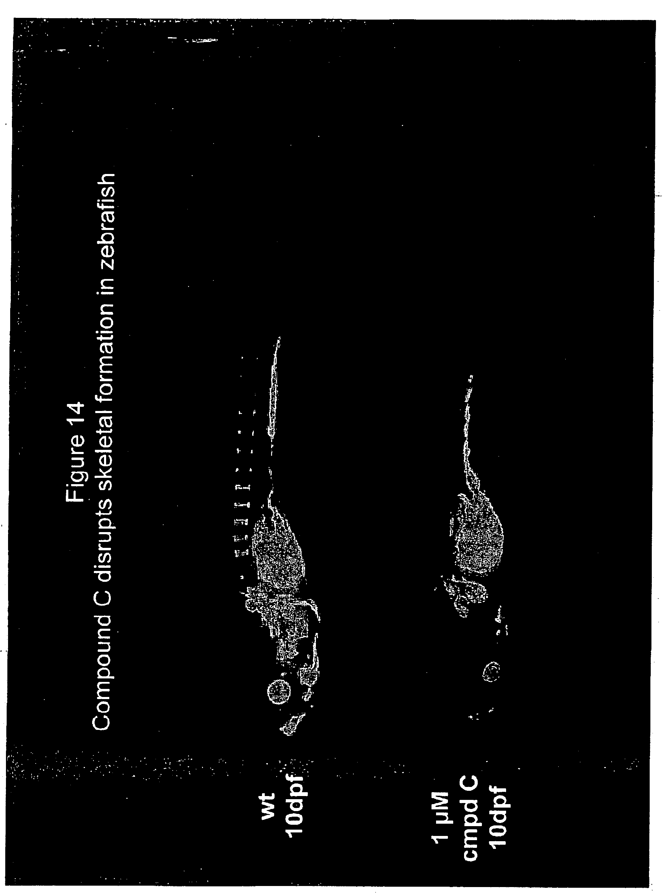

Figure 14 is an image showing that Compound C/Dorsomorphin disrupts vertebral bone mineralization in zebrafish.

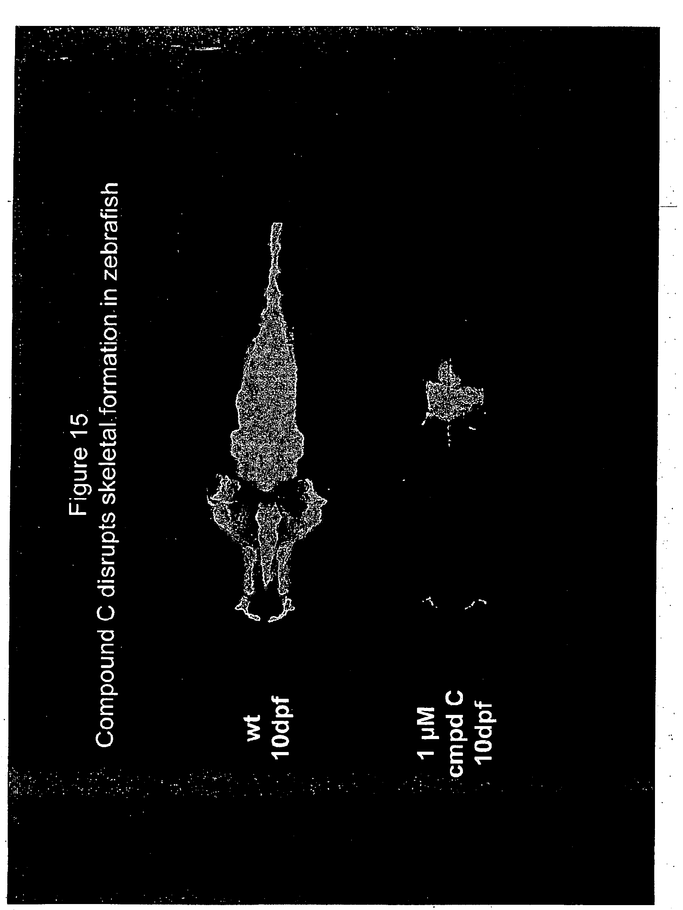

Figure 15 is an image showing that Compound C/Dorsomorphin disrupts craniofacial bone mineralization in zebrafish.
Figure 16 is an image showing that Compound C/Dorsomorphin inhibits bone mineralization without disrupting overall morphology in 10 day post fertilization zebrafish.

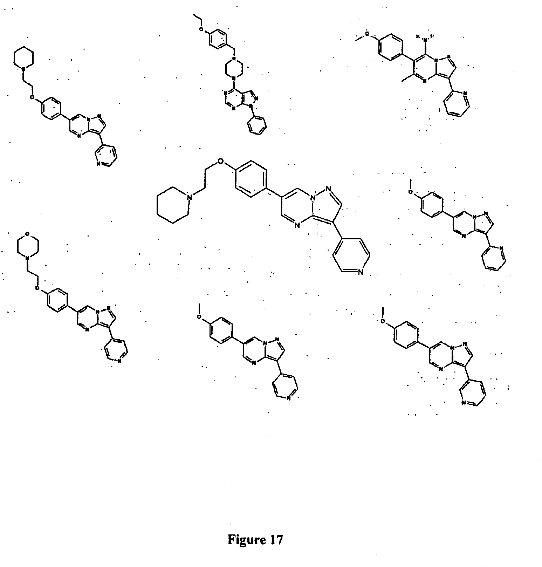

Figure 17 is a schematic illustration of Compound C/Dorsomorphin and analogous structures. Compound C/Dorsomorphin (center) is pictured with several derivative structures, which as a group have been previously described to possess similar profiles for the inhibition of other kinase activities (VEGFR2 or KDR).

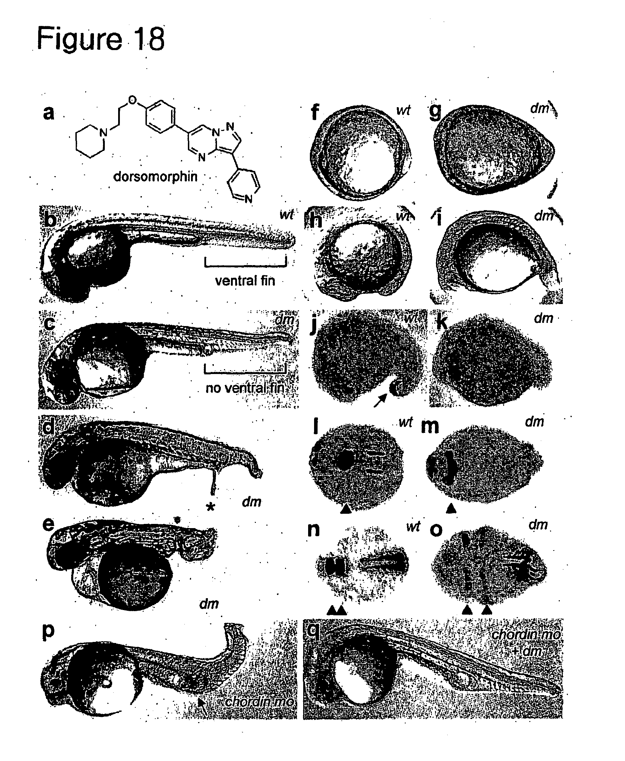

Figure 18 shows that Compound C/Dorsomorphin induces dorsalization in zebrafish embryos. (a) The structure of Compound C/Dorsomorphin is depicted. (b) Vehicle-treated wild-type (*wt)* zebrafish embryos at 36 hpf exhibit normal dorsoventral patterning. Embryos (b-e) are shown on lateral view, anterior left and posterior right. (c) Zebrafish embryos treated with 10 µM Compound C/Dorsomorphin (dm) at 6-8 hpf exhibit mild dorsalization, with loss of the ventral tail fin evident at 36 hpf. (d) Occasional wild-type embryos treated with 10 µM Compound C/Dorsomorphin at 6 hpf developed ectopic tails (*) at 48 hpf. (e) Embryos treated with 10 µM Compound C/Dorsomorphin at 1-2 hpf exhibit severe dorsalization, largely lacking ventrally derived posterior tissue at 48 hpf. (f-g) At the bud stage (f is wild-type), ventral view of embryos treated at 2 hpf with Compound C/Dorsomorphin reveal ovoid shape (g). (h-i, lateral view with anterior to the left) At the 10-somite stage (h is wild-type), embryos treated at 2 hpf with Compound C/Dorsomorphin reveal abnormal tailbud morphology (i). (1-o) *In situ* hybridization of 18-somite stage wild-type embryos shown from the lateral view reveals typical expression of the ventral marker *evel* (arrow) in the distal tail (j), whereas embryos treated with Compound C/Dorsomorphin (10 µM) at 1 hpf do not exhibit *evel* expression in the distal tail (k). (1-m) *In situ* hybridization of 6-somite stage (12 hpf) embryos shown from dorsal view, with anterior left and posterior right. The mid-hindbrain boundary marked by *pax2.1* (arrowheads) expands laterally with Compound C/Dorsomorphin treatment at 1 hpf (m compared with 1, wild-type). Rhombomeres 3 and 5, marked by *Krox20* (arrowheads), and somitic mesoderm, marked by *MyoD,* expand laterally upon Compound C/Dorsomorphin treatment (o compared with n, wild-type). (p) Embryos injected with 1 ng *chordin* morpholino at the single cell stage exhibit hypomorphic head structures with expanded tail structures and intermediate cell mass (arrow) at 36 hpf, consistent with ventralization. (q) In contrast, *chordin* morphant embryos treated at 2 hpf with Compound C/Dorsomorphin (20 µM) exhibit little or no ventralization.

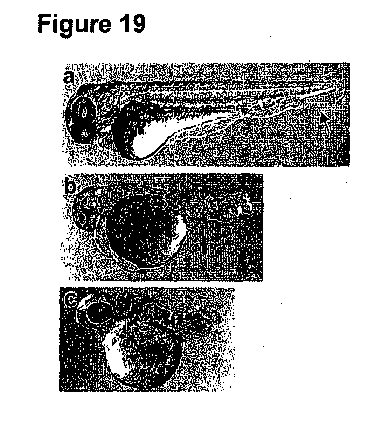

Figure 19 shows that Compound C/Dorsomorphin induces a spectrum of dorsalization phenotypes in zebrafish embryos, which varies with stage of treatment. Varying degrees of dorsalization were observed in zebrafish embryos at forty-eight hours post fertilization (hpf) after treatment with Compound C/Dorsomorphin (10 µM) during periods of early development (0-8 hpf). (a) Embryos treated starting at 6-8 hpf exhibited mild dorsalization, which included, in addition to tailfin defects described in text, partial absence of the ventral tailfin (arrow). (b) Embryos treated at 4-6 hpf exhibited moderate dorsalization defects, characterized by a shortened tail measuring greater than 1/3 the length of the embryo, with a moderately curved distal tail. (c) Embryos treated at 2-4 hpf exhibited severe dorsalization defects characterized by a severe truncated and curved tail measuring less than 1/3 the length of the embryo. The penetrance and severity of dorsalization effects of Compound C/Dorsomorphin treatment at different stages of development is represented in Table 1.

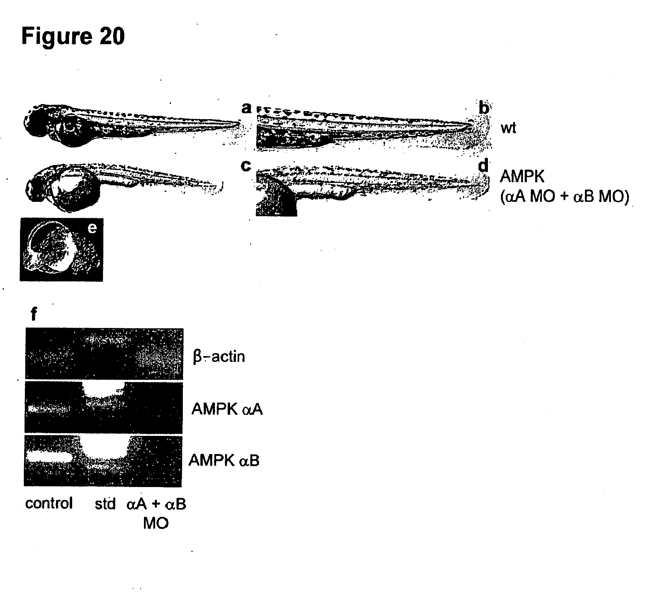

Figure 20 shows that an AMPK α-subunit knockdown does not cause dorsalization in zebrafish embryos. To assess the effects of deficient AMPK activity in zebrafish development, one-cell stage embryos were injected with a splice-targeting morpholinos against the zebrafish orthologs of the catalytic α-subunit of human AMPK, αA (CACCCTGGAAAGTT TTACCTTTGAC (SEQ ID NO: 1)) (exonl-intronl boundary) and αB (TAAATGAACA GACTTAACTCTTCAC (SEQ ID NO: 2)) (exonl-intronl boundary) and examined for phenotypes of 24 hpf. Embryos injected with 8 ng of either morpholino alone did not exhibit any phenotype. (a-e) Injection of Embryos with 4 ng each of both morpholinos together caused mild developmental delay (c-d, versus control a-b), or, in some cases (<8% of embryos), caused necrotic head and tail phenotypes (e). The phenotypes did not resemble dorsalization exhibited by Compound C/Dorsomorphin treatment. (f) The expression of AMPK α-subunits in embryos injected with both AMPK αA + αB morpholinos was examined by RT-PCR for the presence of normally spliced transcripts. These morpholinos efficiently suppressed the expression of AMPK αA (97% knockdown) and AMPK αB (99% knockdown) transcripts, using β-action expression as a control.

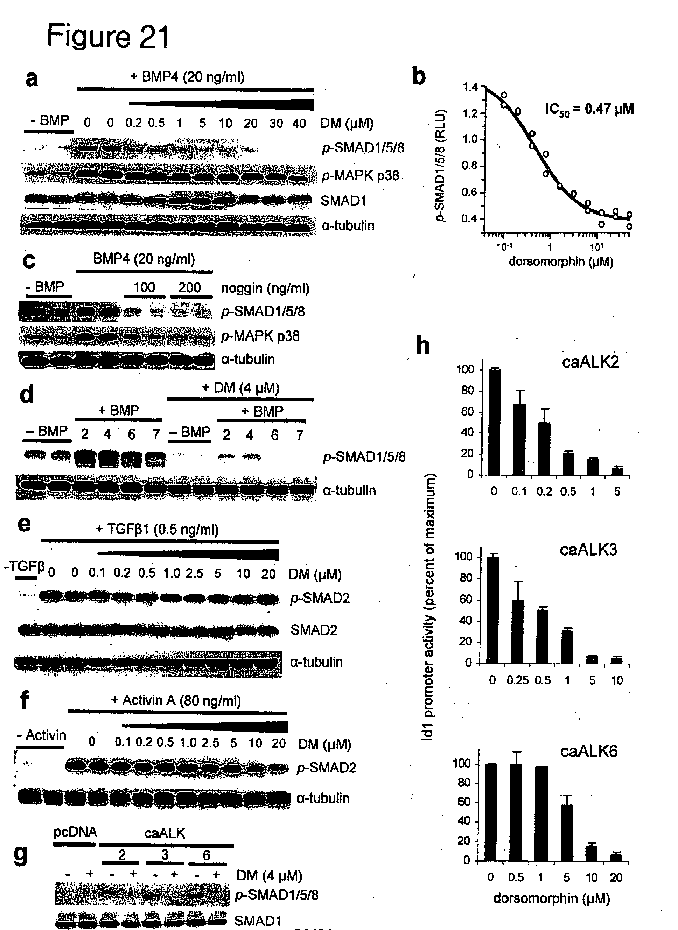

Figure 21 shows that Compound C/Dorsomorphin inhibits BMP-mediated activation of SMAD by inhibiting BMP type I receptor function. (a) Treatment of PASMCs with BMP4 (20 ng/ml) for 30 minutes induced phosphorylation of BR-SMADs (1, 5, and 8) and MAPK p38 detected by immunoblot. Pretreatment with Compound C/Dorsomorphin (DM) at 0.2 - 40 µM for 30 minutes induced a dose-dependent inhibition of BMP4-mediated SMAD1/5/8 phosphorylation, but under the same conditions did not affect BMP4-mediated phosphorylation of MAPK p38. Equivalent protein loading was confirmed by detection of total SMAD1 and α-tubulin. (b) Hill plot of the inhibition of BMP4-stimulated SMAD1/5/8 phosphorylation by incubating PASMCs with Compound C/Dorsomorphin (0.1 - 50 µM), reveals a functional IC₅₀ of 0.47 µM. (c) In contrast to Compound C/Dorsomorphin, pre-treatment of cells with noggin (100 - 200 ng/ml) for 30 minutes inhibited BMP4-mediated phosphorylation of MAPK p38, as well as SMAD1/5/8. (d) Pretreatment with Compound C/Dorsomorphin (4 µM) inhibited the activation of SMAD1/5/8 by BMPs 2, 4, 6, and 7 (10 ng/ml) in PASMCs at 30 minutes and reduced the level of phosphorylated SMAD/1/5/8 below basal levels in cell not treated with BMP. (e) Treatment of PASMCs with TGF-β1 (0.5 ng/ml) induced SMAD2 phosphorylation. Pretreatment with Compound C/Dorsomorphin (0.1 - 20 µM) did not inhibit TGF-β1-mediated activation of SMAD2 at 30 minutes. (f) Treatment of PASMCs with Activin A (80 ng/ml) induced phosphorylation of SMAD2. Compound C/Dorsomorphin inhibited Activin A-mediated activation of SMAD2 only modestly at concentrations ≥10 µM. (g) Transient transfection of BMPR-II-deficient PASMCs with constitutively-active type I receptor cDNA (caALK2, caALK3, or caALK6) increased the levels of phosphorylated SMAD1/5/8 in cell extracts detected by immunoblot. Coincubation with Compound C/Dorsomorphin (10 µM) inhibited the increase in phospho-SMAD1/5/8 seen with caALK transfection. (h) Transient transfection of BMPR-II-deficient PASMCs with caALK2, caALK3, or caALK6 resulted in 5- to 12-fold increases in *Id1* promoter activity (BRE-Luc). *Id1* promoter activity mediated by each of the constitutively active type I receptors was decreased by co-treatment with Compound C/Dorsomorphin (0.1 - 5 µM for caALK2, 0.25-10 µM for caALK3, and 0.5 - 20 µM caALK6) in a dose-dependent manner.

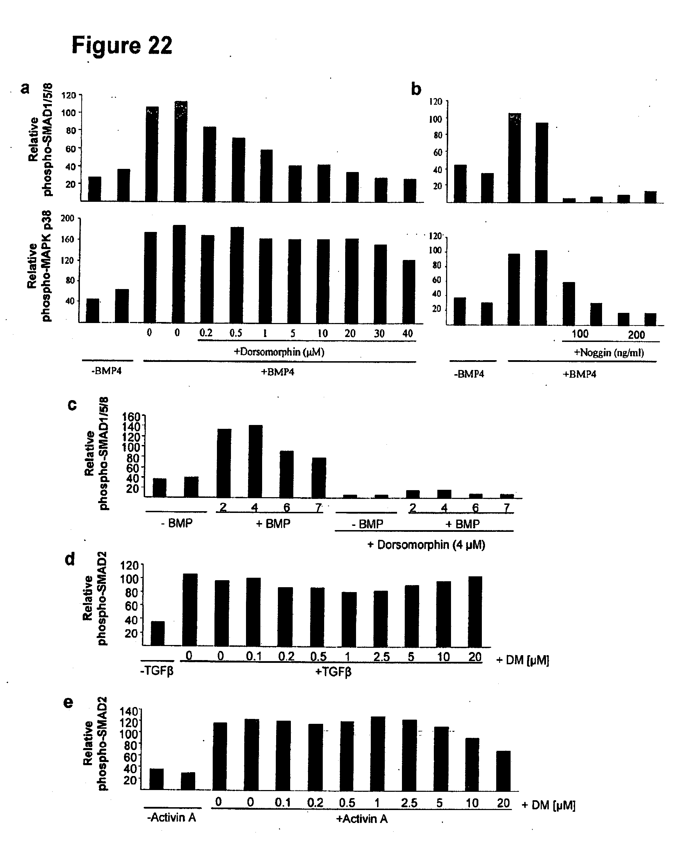

Figure 22 shows that Compound C/Dorsomorphin inhibits BMP-mediated activation of SMAD in preference to MAPK p38, TGF-β and Activin signaling. (a) Treatment of PASMC's with BMP4 (20 ng/ml) for thirty minutes induced phosphorylation of BR-SMAD's (1, 5, and 8) and MAPK p38 detected by immunoblot. Pretreatment with Compound C/Dorsomorphin (DM) at 0.2-40 µM for thirty minutes induced a dose-dependent inhibition of BMP4-mediated SMAD 1/5/8 phosphorylation, but under the same conditions did not affect BMP4-mediated phosphorylation of MAPK p38. Equivalent protein loading was confirmed by detection of α-tubulin. (b) In contrast to Compound C/Dorsomorphin (DM), pretreatment of cells with noggin (100-200 ng/ml) for thirty minutes inhibited BMP4-mediated phosphorylation of MAPK p38, as well as SMAD 1/5/8. (c) Pretreatment with Compound C/Dorsomorphin (4 µM) inhibited the activation of SMAD 1/5/8 by 2, 4, 6, and 7 (10 ng/ml) in PASMC's at thirty minutes and reduced the level of phosphorylated SMAD 1/5/8 below basal levels in cell not treated with BMP. (d) Treatment of PASMC's with TGF-β1 (0.5 ng/ml) induced SMAD2 phosphorylation. Pretreatment with Compound C/Dorsomorphin (0.1-20 µM) did not inhibit TGF-β1-mediated activation of SMAD2 at thirty minutes. (e) Treatment of PASMC's with Activin A (80 ng/ml) induced phosphorylation of SMAD2. Compound C/Darsomorphin inhibited Activin A-mediated activation of SMAD2 only modestly at concentrations ≥10 µM.

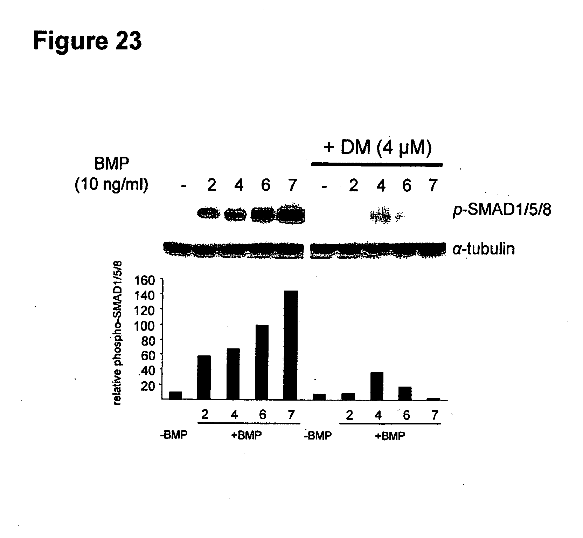

Figure 23 shows the effect of Compound C/Dorsomorphin on BMP signaling in BMPR-II-deficient cells. BMPR-II-deficient PASMC's were treated with various BMP's in the presence or absence of Compound C/Dorsomorphin. Extracts were fractioned by SDS-PAGE and then immunoblotted using antibodies specific for phosphorylated SMAD 1/5/8 and α-tubulin. BMP's 2, 4, 6, and 7 (10 ng/ml) induced the activation of SMAD 1/5/8 at thirty minutes in BMPR-II-deficient PASMC's. Pretreatment with Compound C/Dorsomorphin (4 µM) inhibited the activation of SMAD 1/5/8 by BMP ligands in BMPR-II-deficient cells, demonstrating that inhibition of BMP signaling by Compound C/Dorsomorphin did not require BMPR-II.

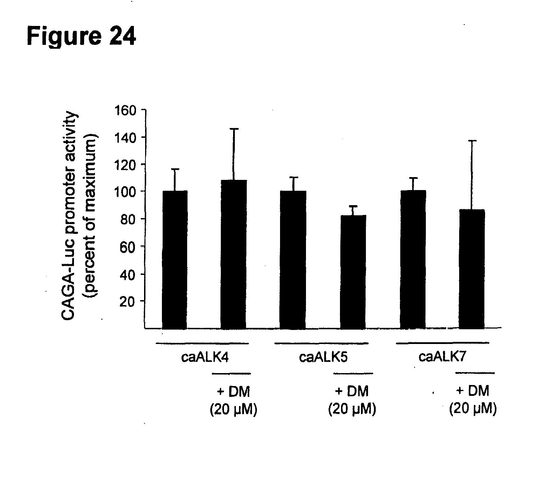

Figure 24 shows that Compound C/Dorsomorphin does not inhibit transcription mediated by constitutively-active type I receptors ALK4, ALKS, and ALK7. Transient transfection of PASMCs with cDNAs encoding constitutively-active type I receptors (caALK4, caALK5, or caALK7) resulted in 2 to 5-fold increases *PAI-1* promoter activity (CAGA-Luc). *PAI-1* promoter activity, expressed as percent of maximum levels of CAGA-Luc activity with cDNA transfection alone, was not decreased by co-treatment with Compound C/Dorsomorphin at concentrations up to 20 µM.

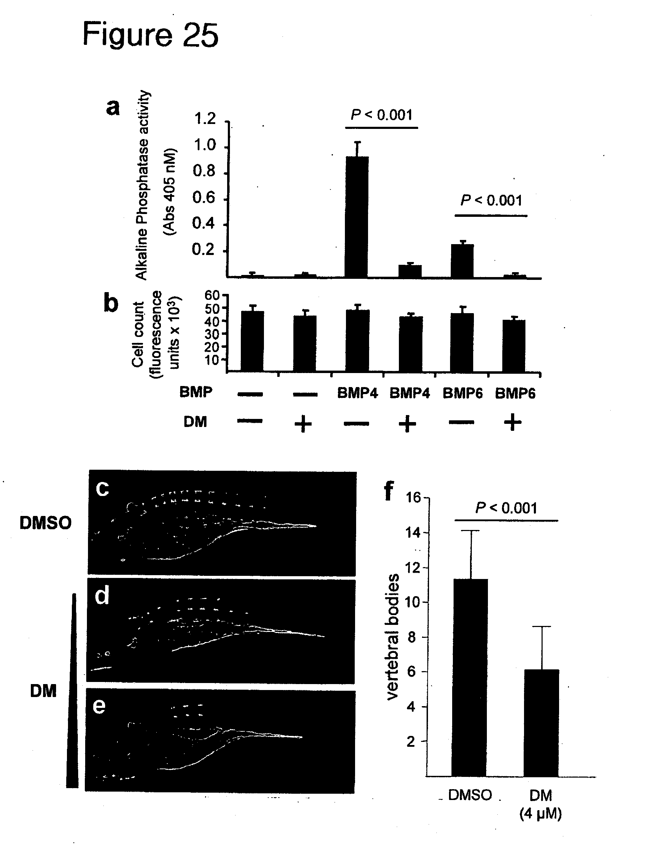

Figure 25 shows that Compound C/Dorsomorphin inhibits osteogenic differentiation in vitro and bone mineralization in vivo. (a) Treatment of C2C12 cells with BMP4 or BMP6 led to expression of alkaline phosphatase activity after 5 days of culture, consistent with osteoblastic differentiation. Pretreatment with Compound C/Dorsomorphin (4 µM) inhibited BMP-mediated induction of alkaline phosphatase activity (n=6 for each condition, results expressed as mean ± S.D). (b) Treatment of C2C12 cells with Compound C/Dorsomorphin did not affect cell count as assayed using DNA-binding dye (CyQuant). (c-e) Visualization of calcified skeletal structures in zebrafish by calcein fluorescence staining at 10 dpf, left lateral view. Wild-type, DMSO-treated fish showed normal vertebral staining of 11-14 segments (c). Treatment of zebrafish at 24 hpf with Compound C/Dorsomorphin (1-4 µM) resulted in viable fish at 10 dpf without evidence of dorsalization. However, a decrease in vertebral segment and craniofacial bone calcification was observed with Compound C/Dorsomorphin-treated vs. vehicle-treated fish (d-e). (f) With Compound C/Dorsomorphin treatment at 4 µM, a significant decrease (*P* < 0.001) in the number of mineralized vertebrae was observed (n=19 for DMSO; n=18 for Compound C/Dorsomorphin treated (DM), results expressed as mean ± S.D.).

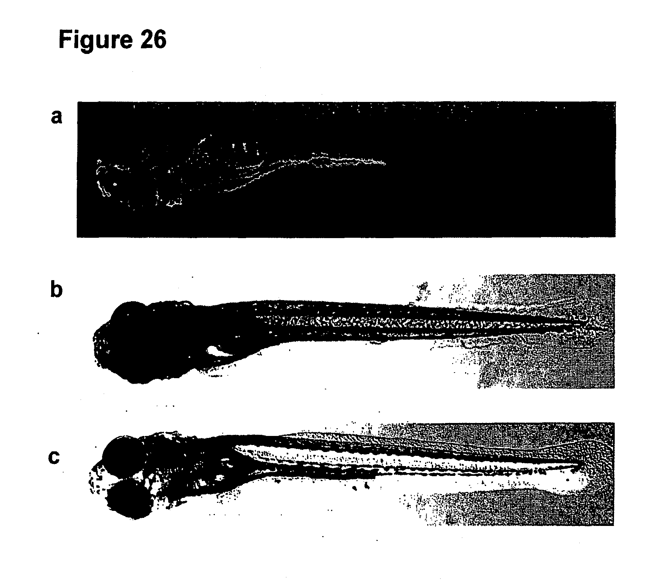

Figure 26 shows that developing zebrafish treated with Compound C/Dorsomorphin exhibit decreased vertebral and craniofacial bone calcification, but are otherwise viable. (a)Treatment of zebrafish with Compound C/Dorsomorphin (1-4 µM) at 24 hpf (after the completion of dorsoventral axis specification) did not result in dorsalization but reduced vertebral segment and craniofacial bone calcification evident at 10 d. (b) However, treated fish appeared morphologically grossly normal and viable by brightfield microscopy, as compared with wild-type control fish (c).

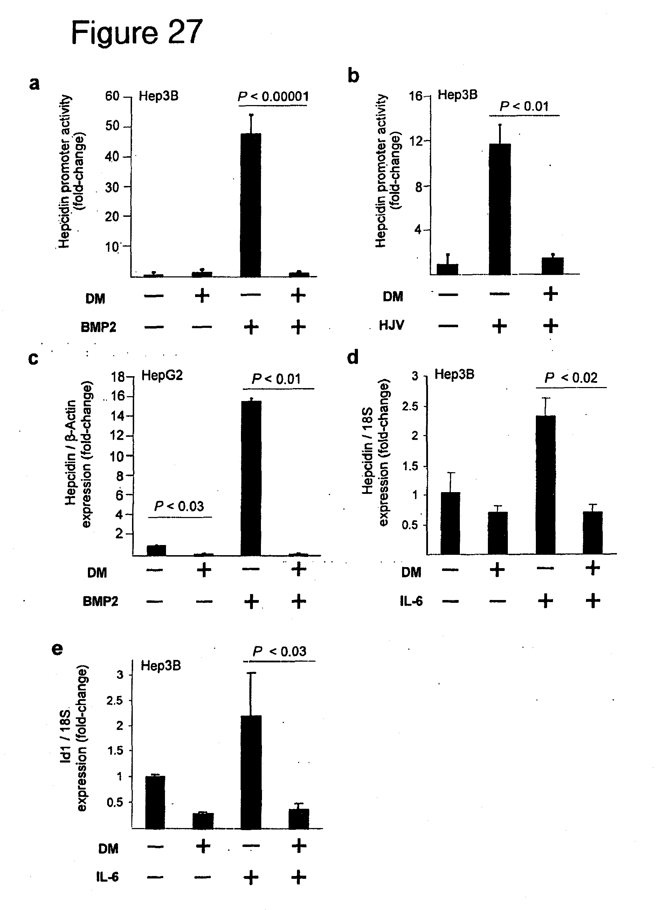

Figure 27 shows that Compound C/Dorsomorphin inhibits BMP- and HJV-induced hepcidin expression in cultured hepatoma-derived cells. (a) Treatment of Hep3B cells with BMP2 (25 ng/ml) increased hepcidin promoter activity nearly 50-fold compared with untreated cells as measured by the hepcidin luciferase reporter assay (Hep-Luc). Co-treatment with Compound C/Dorsomorphin (10 µM) abrogated the induction of hepcidin promoter activity by BMP2 (triplicate measurements, results expressed as mean ± S.D.). (b) Transfection of Hep3B cells with HJV increased hepcidin promoter activity 12-fold. Treatment with Compound C/Dorsomorphin blocked the HJV-mediated increase in hepcidin promoter activity (triplicate measurements, results expressed as mean ± S.D.). (c) Levels of hepcidin mRNA were measured in HepG2 cells by qRT-PCR. Treatment with Compound C/Dorsomorphin (10 µM) reduced basal hepcidin expression by 50-fold. Treatment with BMP2 increased hepcidin by 16-fold over the basal level. Treatment with Compound C/Dorsomorphin (10 µM) in combination with BMP2 reduced the levels of hepcidin mRNA to below the basal level (triplicate measurements, results are expressed as mean ± S.D.) (d-e) In Hep3B cells, levels of hepcidin and Id1 mRNA measured by qRT-PCR were increased in response to IL-6 (100 ng/ml) treatment for 6 hours. Treatment with Compound C/Dorsomorphin (4 µM) alone or in combination with IL-6 reduced the levels of hepcidin and Id1 mRNA to below basal levels, with no effect on expression of the control gene (*18S*) (triplicate measurements, results are expressed as mean ± S.D.).

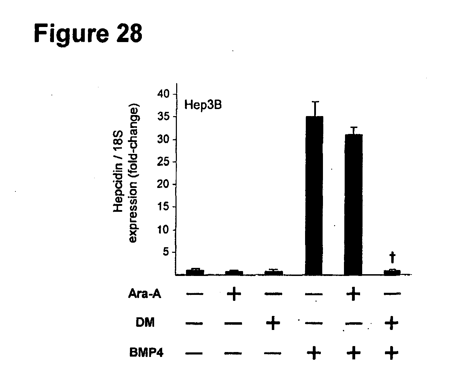

Figure 28 shows that inhibition of AMPK does not inhibit BMP-induced expression of hepcidin. In Hep3B cells, BMP4 (20 ng/ml) increased levels of hepcidin mRNA 35-fold, measured by qRT-PCR after treatment for four hours. Treatment with Ara-A at a concentration known to abrogate AMPK activity (250 µM) or Compound C/Dorsomorphin (DM, 4 µM) alone lowered the baseline level of hepcidin expression by 40 % and 27% respectively. Pretreatment with Ara-A thirty minutes prior to treatment with BMP4 did not significantly affect the ability of BMP4 to induce hepcidin expression, while pretreatment with Compound C/Dorsomorphin completely abrogated the response to BMP4 (results are expressed as mean ± S.D., ^{†}*P* < 0.005 versus BMP4 alone).

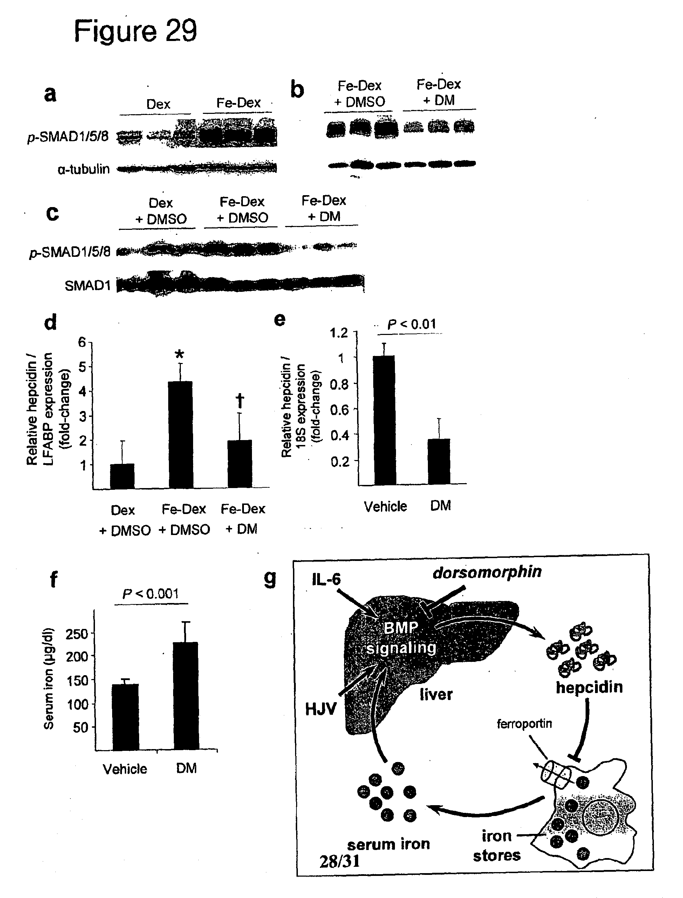

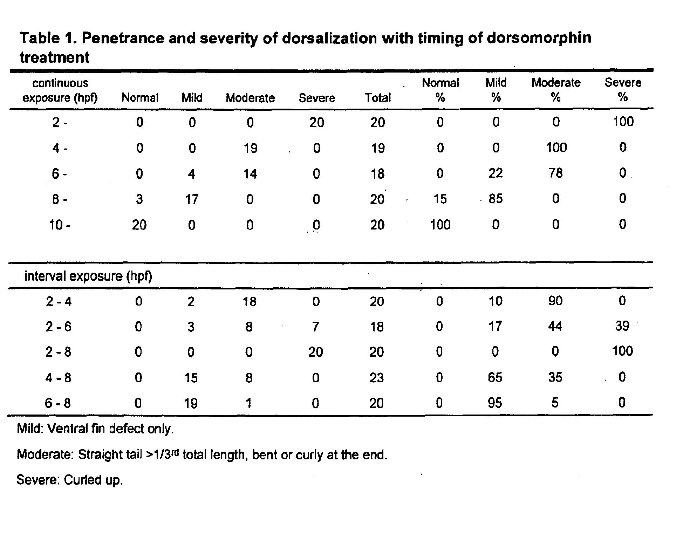

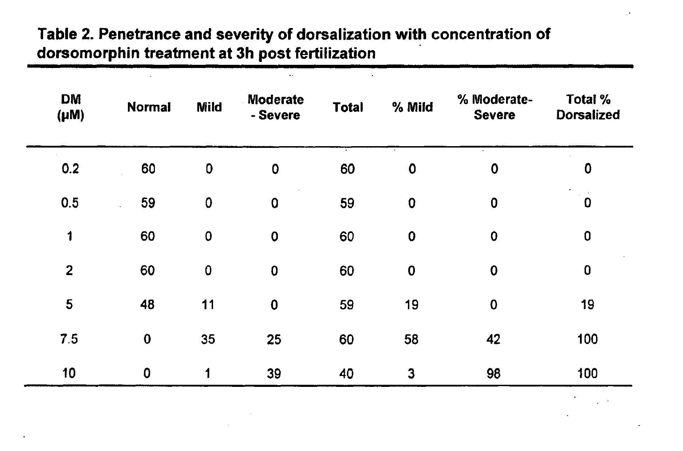

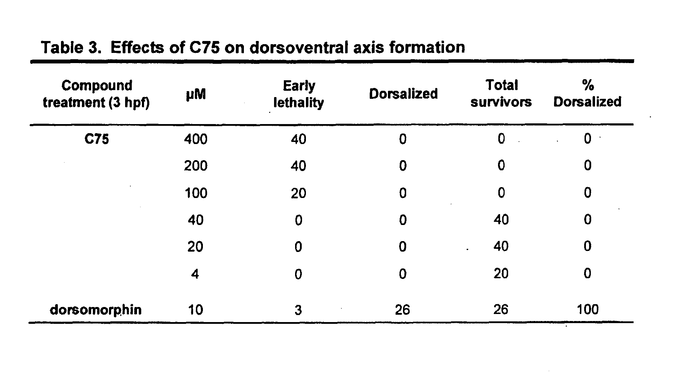

Figure 29 shows that Compound C/Dorsomorphin inhibits iron-mediated hepatic BR-SMAD activation and expression of hepcidin. (a) Intraperitoneal injection of iron-dextran (Fe-Dex) in adult zebrafish resulted in a nearly 3-fold increase in SMAD1/5/8 phosphorylation (normalized to α-tubulin levels) in liver extracts after 1 hour as compared to dextran-injected fish (Dex) (n=3 each group, *P*<0.001). (b) Co-injection of Compound C/Dorsomorphin at 23 µg/g with iron-dextran (Fe-dex + DM) reduced SMAD1/5/8 phosphorylation in zebrafish livers by nearly 3-fold as compared to fish injected with iron-dextran and vehicle (Fe-dex + DMSO) (n=3 in each group, *P*<0.03). (c) Intravenous injection of iron-dextran and vehicle (Fe-Dex + DMSO) in adult mice resulted in a more than 3-fold increase in SMAD1/5/8 phosphorylation in liver extracts after 1 hour as compared to mice injected with dextran and vehicle (Dex + DMSO). Mice injected with iron-dextran and Compound C/Dorsomorphin (Fe-Dex + DM) did not exhibit an increase in hepatic SMAD1/5/8 phosphorylation. (d) Zebrafish injected intraperitoneally with iron-dextran and vehicle (Fe-dex + DMSO) exhibited a more than 4-fold increase in hepatic hepcidin mRNA levels 3 hours post injection, (normalized to liver fatty acid binding protein mRNA levels) as compared to fish injected with dextran and vehicle (Dex + DMSO) (n=4 each group, * *P*<0.01, ANOVA). Co-injection of iron-dextran with 23 µg/g Compound C/Dorsomorphin (Fe-dex + DM) reduced hepcidin mRNA levels compared to iron-dextran-treated fish († *P* <0.02, ANOVA) to a level not significantly different from dextran-treated fish. (e) In C57BL/6 mice, single tail vein injection of Compound C/Dorsomorphin (10 mg/kg) reduced liver hepcidin mRNA levels to one-third of that in controls after 6 hours (n=6 each group, *P*<0.01, results are expressed as mean ± S.D). (f) In C57BL/6 mice, two intraperitoneal injections of Compound C/Dorsomorphin (10 mg/kg) 12 hours apart increased serum iron levels measured 24 hours after first injection by over 60%. (n=8 wild type, n=7 Compound C/Dorsomorphin, *P*<0.001, results are expressed as mean ± S.D). Panels a, b, and f are representative of three independent experiments each, while panels c-e are representative of two independent experiments each. (g) Proposed model for the role of BMP signaling in iron homeostasis. High serum iron levels, HJV, and IL-6 all require BMP signaling in the liver to upregulate transcription of hepcidin. Increased hepcidin levels cause ferroportin degradation and a decrease in iron export from intracellular stores to the blood. Inhibition of BMP signaling by Compound C/Dorsomorphin decreases basal hepcidin transcription, resulting in higher serum iron levels. Thus, BMP signaling is essential for the feedback regulation of iron in the serum.

Table 1 - Treatment with Compound C/Dorsomorphin (10 µM) during different periods of early zebrafish development elicited varying degrees of dorsalization with varying degrees of penetrance. Continuous exposure starting earlier in development causes more severe dorsalization defects. Zebrafish embryos were exposed continuously to Compound C/Dorsomorphin starting at various times after fertilization and assessed for phenotypes as defined at 48 hpf. Compound C/Dorsomorphin treatment starting at 2 hpf elicited severe dorsalization with high penetrance, while Compound C/Dorsomorphin at 4 - 6 hpf elicited mild to moderate dorsalization with high penetrance. Compound C/Dorsomorphin treatment starting at 8 hpf elicited mild dorsalization with 85% penetrance, whereas treatment at 10 hpf and beyond did not induce dorsalization. No toxicity was observed with this concentration of Compound C/Dorsomorphin. Interval exposure: Zebrafish embryos were exposed to Compound C/Dorsomorphin starting at various times post fertilization, then transferred to fresh media after an interval of time to test the effects of interval exposure. Compound C/Dorsomorphin starting at 2 hpf elicited progressively more severe dorsalization when exposure was continued to 4, 6, or 8 hpf, with exposure from 2 - 8 hpf yielding identical severity and penetrance (100% severely dorsalized) as embryos exposed continuously to Compound C/Dorsomorphin after 2 hpf. Exposure to compound from 4 - 8 and 6 - 8 hpf both elicited mild to moderate dorsalization. The effect of Compound C/Dorsomorphin upon dorsoventral axis formation was therefore restricted to before 10 hpf and was more penetrant and severe with earlier and longer exposure during this period of susceptibility.
Table 2 - Treatment of zebrafish with varying concentrations of Compound C/Dorsomorphin (0 - 10 µM) during early embryogenesis elicited dorsalization with varying degrees of penetrance and severity. Zebrafish embryos were exposed continuously to various concentrations of Compound C/Dorsomorphin starting at 3 hpf and assessed for phenotypes at 48 hpf, as defined in Figure 19. Concentrations of Compound C/Dorsomorphin ≤ 2 µM did not elicit dorsalization under these conditions, while concentrations ranging from 5 to 10 µM elicited progressively more severe dorsalization in a progressively higher percentage of embryos, with 10 µM inducing moderate or severe dorsalization in nearly all embryos examined.
Table 3 - Compound C/Dorsomorphin is a known inhibitor of AMP kinase (AMPK). To exclude the possibility that the effects of Compound C/Dorsomorphin were attributable to modulation of AMPK, we tested the effect of C75, an inhibitor of fatty acid synthase known to suppress AMPK activity, on dorsoventral axis formation in zebrafish embryos. Treatment of zebrafish embryos with C75 starting at 3 hpf caused nonspecific toxicity at concentrations ≥ 25 µg/ml without evidence of dorsalization at any concentration tested (1 - 100 µg/ml). In contrast, treatment with Compound C/Dorsomorphin induced dorsalization with high penetrance and minimal toxicity under the same conditions.

### Detailed Description

The invention is based on (i) the development of a whole-animal (e.g., zebrafish) screen, (ii) the development of a cell-based ELISA screen, and (iii) the identification of a novel class of BMP inhibitors. The zebrafish and cell-based ELISA screens can be used to identify compounds that modulate BMP or TGF-β signaling pathways. As is discussed further below, the novel class of BMP inhibitors is exemplified by Compound C/Dorsomorphin specifically inhibits BMP signaling, while having little impact on TGF-β signaling. Additional examples of such compounds are shown in Figure 17.

The invention also provides methods of treating and preventing diseases and conditions associated with altered BMP or TGF-β signaling, which involve administration of compounds identified using screening methods such as those described herein (e.g., Compound C/Dorsomorphin). Thus, for example, the invention includes methods of treating patients having diseases or conditions that could benefit by inhibition of BMP signaling, which involve administration of dorsomorpin or related compounds. Also included in the invention are methods of manipulating cultured cells to change or maintain their phenotypes. For example, a compound such as Compound C/Dorsomorphin may be used to prevent differentiation of stem cells (e.g., embryonic stem cells or adult stem cells) or, under other conditions, used to differentiate stem cells along a desired lineage.

The invention is described further, as follows.

### Therapeutic Methods

Compounds that modulate BMP or TGF-β signaling pathways, such as compounds identified using the screening methods described herein, can be used in methods of treating or preventing diseases or conditions that would benefit from modulation of these signaling pathways. Examples of diseases and conditions that can be treated by modulation of these pathways include those described below.

We have shown that deficiency of bone morphogenic protein receptor 2 (BMPR2) can result in increased BMP signaling (Yu et al., J. Biol. Chem. 280:24443-24450, 2005), which may cause cardiopulmonary abnormalities, such as pulmonary hypertension. Inhibition of BMP signaling, according to the invention, can thus be used to prevent or treat cardiopulmonary diseases and conditions. For example, the methods of the invention can be used to prevent the development of familial and sporadic primary pulmonary hypertension in unaffected individuals, such as those with a genetic predisposition for pulmonary hypertension, or reverse, slow, or prevent the progression of disease in affected individuals.

In certain inborn cardiac valvular malformations, excessive BMP stimulation may cause excessive or insufficient tissue growth in intraventricular or valvular precursors (Yu et al., J. Biol. Chem. 280:24443-24450, 2005; Delot et al., Development 130:209-220, 2003). Inhibition of BMP signaling, according to the invention, can be carried out to correct the excessive BMP signal, and thus restore normal valvular or cardiac tissue growth.

Excessive BMP signaling, either due to loss of BMP type II receptor expression, or to overexpression of BMPs themselves, may contribute to oncogenesis of certain solid tumors, including breast carcinomas, prostate carcinomas, and renal cell carcinomas (Yu et al., J. Biol. Chem. 280:24443-24450, 2005; Waite et al., Nat. Rev. Genet. 4:763-773, 2003; Alarmo et al., Genes Chromosomes Cancer 45:411-419, 2006; Kim et al., Cancer Res. 60:2840-2844, 2000; Kim et al., Oncogene 23:7651-7659, 2004; Kim et al., Clin. Canc. Res. 9:6046-6051, 2003). TGF-β and BMPs can attenuate the inflammatory or immune response (Choi KC et al, Nat Immunology, 7:1057-65, 2006) which may impair an individuals ability to fight infections (i.e., viral, bacterial, fungal, parastici, or tuberculosis). Inhibitors of BMP or TGF-β signaling may thus augment the inflammatory or immune response. Inhibition of BMP signaling, according to the invention, can be carried out to slow or arrest the growth of such tumor cells (as well as other tumor cell types) for clinical benefit, either as adjunctive or primary chemotherapy. Also, BMP inhibitors can be used to interfere with the bone metastatic properties of certain types of cancers (e.g., adenocarcinoma, such as prostate and breast carcinomas). In addition, inhibition of BMP signaling can be used to ameliorate osteoblastic activity in tumors that form bone, such as osteosarcomas (as adjunctive or primary chemotherapy). Further, BMP inhibitors can be used to inhibit osteoclastic activity (also regulated by BMPs through the action of its target gene RANKL), which is pathologically increased in conditions such as multiple myeloma, and other bone targeted tumors.

In another example, inhibition of BMP signaling can be carried out to ameliorate pathologic bone formation/bone fusion in inflammatory disorders, such as ankylosing spondylitis or other "seronegative" spondyloarthropathies.

In a further example, BMP signaling can be inhibited, according to the invention, for use in treating and preventing iron deficiency. In particular, BMP is a positive regulator of hepcidin, which blocks iron absorption. Inhibiting BMP can thus be used to increase iron absorption and constitute a treatment for anemia and iron deficiency anemia. The therapeutic methods of the invention can therefore be used to treat patients with anemia, such as patients with incomplete or slow responses to iron replacement and/or epogen therapy.

Specific BMP modulation, according to the invention, can also be employed to direct the differentiation of stem cells (e.g., embryonic stem cells) or tissue progenitor cells towards specific lineages for therapeutic application (Park et al., Development 131:2749-2762, 2004; Pashmforoush et al., Cell 117:373-386, 2004). As an example, a compound found to activate BMP signaling can be used to promote hemangioblast formation for use in methods of promoting growth of the vasculature.

In another example, the manipulation of BMP signals with a small molecule inhibitor in embryonic stem cell populations can be used to promote self-renewal or regulating differentiation, as this process is regulated by the balance between BMP and FGF signals in human embryonic stem cells, and is promoted by the activity of BMP in combination with LIF in murine embryonic stem cells.

The ectopic formation of bone or calcification mediated by BMPs, such as in pathologic vascular or cardiac valve calcification, atherosclerosis, and renal osteodystrophy, can be inhibited by BMP inhibitors. In the case of prosthetic vascular grafts or prosthetic cardiac valves, this can be carried out by, for example, systemic administration or direct incorporation into prosthesis materials.

A rare autosomal dominant disease of heredity known as Fibrodysplasia Ossificans Progressiva (FOP) has recently be found to arise from mutations in the ALK2 receptor, which render the receptor kinase constitutively active (Shore et al., Nat. Genet. 38:525-527, 2006). This disease manifests in early childhood as inappropriate bone formation, both spontaneous and secondary to trauma, causing excessive mineralization of a "second skeleton" in parallel with the normal axial and appendicular skeletal framework. A specific inhibitor of BMP signaling can be used to prevent excessive bone formation or induce regression of pathologic bone formation. Undesirable or pathologic bone formation can be inhibited, and bone regression can be achieved, by systemic or local administration of a BMP inhibitor.

TGF-β is a central mediator of fibrosis in human fibrotic diseases involving the liver, kidneys, and lungs. Specific TGF-β inhibitors can thus be employed to reduce fibrosis in these and other disease contexts.

Valvular and aortic abnormalities exhibited by Marfan's syndrome patients are thought to result from pathologic upregulation of TGF-β signaling. Therefore, specific TGF-β inhibitors can be used to treat or prevent cardiovascular complications of Marfan's syndrome.

Aberrant TGF-β/BMP signaling has also been implicated in human diseases such as aortic aneurysm, atherosclerosis, and hereditary hemorrhagic telangectasia syndrome. Thus, compounds that modulate these signaling pathways can be used in the treatment and prevention of these and related diseases and conditions as well.

In addition to inhibiting BMP or TGF-β signaling, compounds can be identified according to the invention that activate these pathways. Compounds that activate BMP signaling can be used in the treatment of, for example, diseases or conditions that would benefit by the promotion of bone growth or development. Thus, such compounds can be used to treat osteoporosis and osteopenia, as well as to promote the healing of fractured bones. In addition, compounds that activate BMP signaling can be used to treat acute tubular necrosis in kidney ischemia reperfusion injury, to ameliorate stroke reperfusion injury, and to ameliorate inflammatory bowel disease.

Compounds that modulate BMP or TGF-β signaling pathways can be used to treat subjects (e.g., humans, domestic pets, livestock, or other animals) by use of dosages and administration regimens that are determined to be appropriate by those of skill in the art, and these parameters may vary depending on, for example, the type and extent of the disorder treated, the overall health status of the subject, the therapeutic index of the compound, and the route of administration. Standard clinical trials can be used to optimize the dose and dosing frequency for any particular pharmaceutical composition of the invention. Exemplary routes of administration that can be used include oral, parenteral, intravenous, intra-arterial, subcutaneous, intramuscular, topical, intracranial, intraorbital, ophthalmic, intraventricular, intracapsular, intraspinal, intracisternal, intraperitoneal, intranasal, aerosol, or administration by suppository. Methods for making formulations that can be used in the invention are well known in the art and can be found, for example, in Remington: The Science and Practice of Pharmacy (20th edition, Ed., A.R. Gennaro), Lippincott Williams & Wilkins, 2000.

In addition to being administered to patients in therapeutic methods, the compounds described herein can also be used to treat cells and tissues, as well as structural materials to be implanted into patients (see above), ex vivo. For example, the compounds can be used to treat explanted tissues that may be used, for example, in transplantation.

### Mechanistic and Experimental Studies

The invention also includes the use of the compounds described herein as research tools. For example, the compounds can be used in the development of other compounds having activities such as those described herein. In these methods, the compounds described herein can be used as models for the design of further compounds or as controls in testing other compounds.

Further, the invention includes use of the compounds described herein (or other BMP or TGF-β signaling modifiers) in the study of these signaling processes. In one example, BMP and TGF-β inhibitors can be used to reduce BMP signaling in cellular or animal models, to correct excessive BMP signaling and rescue disease phenotype, or to induce deficient BMP signaling to model other disease phenotypes. For example, administration of a BMP-selective inhibitor to developing animals may perturb aspects of cardiovascular development and vasculogenesis which critically depend on BMP signaling, and thus recapitulate congenital cardiac disease or pulmonary vascular disease in which abnormal or deficient function of BMP signaling pathways is implicated, providing valuable disease models and insights (Waite et al., Nat. Rev. Genet. 4:763-773, 2003; Delot et al., Development 130:209-220, 2003). In another example, compounds of the invention, such as Compound C/Dorsomorphin, can be used to distinguish between SMAD-dependent and SMAD-independent mechanisms of BMP action, in vivo and in vitro.

### Screening Methods

As is noted above, the invention provides methods (e.g., high throughput methods) for identifying compounds (e.g., small organic or inorganic molecules) specific for modulating either BMP or TGF-β signaling. BMP and TGF-β activate distinct subsets of receptors and downstream mediators, and also have diverse and sometimes opposing functions. However, as discussed above, due to overall structural similarities of the TGF-β/BMP receptors, identification of small molecules with specificity for either TGF-β or BMP pathways by traditional biochemical methods has been challenging. The methods of the present invention, as described below, overcome these challenges and enable the identification of specific modulators (antagonists or agonists).

### Whole Animal Screening Methods

In zebrafish, the TGF-β and BMP-dependent signaling cascades play distinct roles in embryonic patterning. For instance, BMP signaling is required to establish ventral cell fate, and the loss of BMP signaling results in dorsalization of embryonic body axis. In contrast, nodal (TGF-β) signaling is required in the dorsal mesoderm to induce ventral brain and dorsal mesendoderm, with loss of such signaling leading to cyclopia and/or the formation of a relatively small head. The distinct phenotypes produced by inhibition of either signaling pathway in the zebrafish embryo facilitate the identification of compounds that inhibit either pathway, according to the invention, by phenotypic analysis of zebrafish embryo contacted with candidate compounds.

Based on these principles, we have identified a compound (Compound C/Dorsomorphin) that preferentially inhibits BMP, but not the TGF-β, signaling. This compound is described further below. In further support of the specificity provided by this screening method, we find that SB-431542, a known specific inhibitor of the TGF-β/activin/nodal branch, produces phenotypes consistent with the loss of nodal signaling, such cyclopia, but not dorsalization. Selectivity of each compound for either the BMP or TGF-β signaling, according to the invention, can determine whether it causes one or the other, but not both, phenotypes. The assay is also highly sensitive to non-specific toxicity, which typically results in less stereotyped abnormalities or death. Thus, the phenotype-based, whole-animal screening platform of the invention can be used for the discovery and lead optimization of specific, selective inhibitors of BMP or TGF-β signaling, while simultaneously selecting against toxic compounds.

According to one example of the whole animal-based screening methods of the invention, 1-2 hour old zebrafish embryos are arrayed in the wells of 96-well plates, three embryos per well. A chemical library, which is comprised of tens of thousands of diverse small molecules, is then pin-transferred into the wells at a final concentration of ~2 to 10 uM. Embryos are observed over the course of next 24-48 hours and scored under a dissecting microscope for phenotype consistent with loss of BMP signaling (dorsalized embryos, Figure 1) or with the loss of nodal signaling (cyclopia, Figure 2).

The dorsalization of axis and the loss of nodal signaling in treated embryos can be confirmed using in situ hybridization with region-specific molecular markers. For example, in dorsalized embryos, the dorsal markers Krox20 and Pax2.1 are greatly expanded, whereas the ventrolateral marker, evel, is reduced. Moreover, loss of dorsal mesendoderm and ventral neural structures due to nodal signaling inhibition can be confirmed by the respective loss of goosecoid and no tail expression.

### Cell Culture Screening Methods

Compounds that modulate BMP or TGF-β signaling pathways can also be identified and characterized using cultured cells. Inhibition of the BMP pathway will prevent the induction of SMAD1/5/8 phosphorylation by BMP4, whereas inhibition of TGF-β pathway will block induction of SMAD2 phosphorylation by TGF-β. Conversely, activation of the BMP pathway will lead to increased SMAD1/5/8 phosphorylation by BMP4, whereas activation of the TGF-β pathway will lead to increased SMAD2 phosphorylation by TGF-β. This technique can be used to confirm the specificity of candidate compounds toward BMP or TGF-β.

In one example, the cell-based assay of the invention is an enzyme linked immunosorbent assay (ELISA) in which activation of SMAD proteins (SMAD2 or SMAD1/5/8) is measured. Intracellular SMAD proteins exist in equilibrium between phosphorylated (active) and non-phosphorylated (inactive) states, with the activated protein being retained in the nucleus (Schmierer et al., Mol. Cell. Biol. 25:9845-9858, 2005). As discussed above, SMAD proteins are phosphorylated by heteromeric complexes of type I and type II BMP or TGF-β serine-threonine kinase receptors when engaged by ligands. The principal effects of these ligands are generally attributable to gene transcription from the activation of specific SMADs. Phosphorylated SMADs can be detected in whole cell extracts using commercially available anti-phospho-protein antibodies with high specificity by Western blotting (Yu et al., J. Biol. Chem. 280:24443-24450, 2005).

In an improved method provided by the invention, phosphorylated SMADs are detected in whole cells with high sensitivity and specificity by immobilizing and rendering the phospho-SMAD antigens accessible to antibody. Traditional fixation with 1% to 8% paraformaldehyde solution followed by permeabilization with methanol, or a solution of dilute detergent such as Triton-X100, yields unacceptable results, with the signal being only 1.2 to 1.3 times the background, if it is detected at all (while Western blot these signal-to-noise ratios would be 10 to 20). This problem may be the result of a fixation process that has failed to cross-link and immobilize the intranuclear soluble protein, which subsequently diffuses, incomplete permeabilization of the nucleus, the creation of neoepitopes by the fixation, or rendering of the antigen as cryptic by the fixation, due to the massive alteration of surface charge characteristics seen with paraformaldehyde.

The present invention overcomes the fixation problem noted above by first thoroughly permeabilizing plasma and nuclear membranes, and precipitating proteins while retaining cytoskeletal structure using ice cold methanol for 10 to 15 minutes, and then cross-linking precipitated proteins with a glutaraldehyde solution of 0.25% to 2% in saline for 10 to 15 minutes at room temperature. This alone achieves an improvement in signal-to-noise from 1.2 to 3, which is a vast improvement but still is only barely sufficient for high-throughput screening, due to a high background. The glutaraldehyde fixative is bifunctional, causing many amino nitrogens on target proteins to be substituted with a terminal aldehyde that does not react with neighboring amines, due to lack of proximity. This aldehyde is ultimately oxidized to a carboxylic acid, which is opposite in charge to the original amine nitrogen, thus altering the surface charge characteristics. To prevent this modification, the glutaraldehyde is instead "quenched" with a bifunctional amine molecule, such as lysine, ethylenediamine, or phenylenediamine, at a concentration of 25-200 mM at pH 8-10. These molecules react with the free aldehyde on monosubstituted glutaraldehyde molecules to form amide linkages, leaving terminal amines or amine/carboxylic acid zwitterions, either of which is closer to the original charge configuration. We have found that this step further improves the signal-to-noise ratio to the range of 7 to 10, which is a much more comfortable margin for high-throughput applications.

We also have employed mutant cells in this assay, which lack the BMP type II receptor. Consistent with results from Western blotting and gene expression studies (Yu et al., J. Biol. Chem. 280:24443-24450, 2005), we find that these mutant cells have substantially increased sensitivity to BMP7 and similar ligands in this assay (Figure 3). This finding has been explained by the use of alternate BMP receptors such as the activin type II receptor, the activity of which is intact and may be enhanced in the absence of the BMP type II receptor. Recombinant BMP7 has been found to have useful in vivo activities in several small animal disease models. The application of this high throughput assay using these mutant cells in comparison to wild-type cells can be used to reveal specific molecules that target this alternative signaling pathway, and function as specific BMP7 mimics in a clinically useful manner.

This assay has been used successfully to identify a compound that preferentially inhibits the activation of the SMAD pathway by BMP ligands, demonstrating its utility as a screening assay for the identification of small molecules that modulate the BMP signaling pathway. Using versions of this assay that detect TGF-β-mediated SMAD2 phosphorylation, and BMP-mediated SMAD1/5/8 phosphorylation, we show that Compound C/Dorsomorphin preferentially inhibits the serine-threonine kinase activity of the BMP receptor versus that of the TGF-β receptor system. This assay therefore aided in the identification of a compound with a novel activity that has not been previously ascribed to any other small molecule (preferential inhibition of BMP signaling) and provided quantitative data on the specificity and potency (IC₅₀) for each of these two pathways (Figure 4).

The assay described above provides several advantages. For example, it does not require stable transfection of cells with a reporter construct, and thus can be carried out with primary cells of early passage without drug selection on virtually any cell line. It also provides improved specificity. In particular, by measuring the activation of proximal signal transduction molecules, the SMADs, over a short time course, this assay avoids the inadvertent detection of activities or compounds that modulate unrelated pathways, cause cell toxicity, or otherwise impair transcription or translation. We find that SMAD phosphorylation measured after 5 to 60 minutes of exposure is modulated only by specific ligands (i.e., BMPs and TGF-β) or their inhibitors (i.e., noggin and follistatin) and is not modulated by non-specific toxicity or activation (e.g., heat shock, osmotic shock, non-specific and specific MAP kinase activation or inhibition, and inhibition of gene transcription or protein translation). In contrast, assays that rely on the expression of a reporter construct may be easily perturbed by any of these factors. These limitations are particularly troubling if one seeks to identify antagonists of BMP or TGF-β signaling, since reporter expression methods may tend to identify many non-specific antagonists. The identification of specific BMP or TGF-β agonists is also limited in reporter expression methods, since gene transcription is not a strict function of SMADs, but also of transcriptional co-repressors and co-activators, the activity of which may be modulated by compounds in a pathway independent manner.

Additional advantages provided by the assay described above concerns its rapidity, as results can be obtained within 3 hours after cell stimulation, however greater sensitivity may be achieved over longer time frames (e.g., 12 hours). Further, the assay is cost effective, as it requires fewer and relatively inexpensive reagents than other assay formats, making it particularly suitable for high throughput applications.

The assay can also be used in research as an assay for the measurement of biologic modulators of BMP or TGF-β signaling pathways in vitro, using any cell type. Further, it can be used as a platform for large throughput screening of compounds with therapeutic potential, which act as BMP agonists or antagonists, or TGF-β agonists or antagonists. In addition, the assay can be used as a diagnostic tool for detecting aberrant responses in ex vivo tissues to BMP or TGF-β signals that may be clinically relevant, and as a diagnostic tool that may yield prognostic or clinicopathologic information from tissue samples from patients, for example, in the response of tumor tissues to BMP or TGF-β. Further, the assay can be used in the context of ELISA analysis of other intranuclear proteins.

### Example 1

Using screening methods such as those described above, we have identified a small molecule that preferentially inhibits the activation of SMADs by bone morphogenetic protein ligands. This molecule, Compound C/Dorsomorphin, was previously described as an inhibitor of the activation of AMP kinase signaling pathway (Zhou et al., J. Clin. Invest. 108:1167-1174, 2001; Calbiochem Catalog #171260; Sigma Catalog #P5499; Figure 5).

Results of experiments showing the effects of Compound C/Dorsomorphin on BMP and TGF-β activation are shown in Figures 6 and 7. As is shown in Figure 6, we have found that Compound C/Dorsomorphin inhibits BMP signaling, as shown by activation of SMADs 1, 5, and 8 by BMP4, with an IC₅₀ of 0.5 uM (Figure 6). We also have found that the compound does not affect the TGF-β signaling pathway (Figure 7). Compound C/Dorsomorphin was previously described to inhibit AMP kinase activity with an IC₅₀ of approximately 15-30 uM (Zhou et al., J. Clin. Invest. 108:1167-1174, 2001). Compound C/Dorsomorphin thus inhibits BMP signaling with > 15 to 60-fold specificity vs. AMP kinase signaling pathways. Compound C/Dorsomorphin does not exhibit significant inhibition of activation of MAP kinase p38 mediated by BMP4 (Figure 8). Previous reports have shown that Compound C/Dorsomorphin also does not exhibit significant inhibition of activation of PI-3 kinase, JAK, STAT, PKA, PKC, and various other pathways by their respective ligands.

We also find that Compound C/Dorsomorphin exhibits inhibition of BMP signaling in vivo, in a specific manner with minimal non-specific toxicity. The transgenic overexpression of BMP ligands or their endogenous inhibitors is known to modify the development of early (0-48 h post fertilization, or hpf) zebrafish embryos by altering dorso-ventral patterning (Figure 9A-C). Overexpression of BMPs in the zebrafish embryo causes exaggerated development of ventral structures with decreased development of anterior structures, known as ventralization (Nguyen et al., Dev. Biol. 199:93-110, 1998). Overexpression of noggin or other BMP antagonists causes dorsalization, ranging in severity from decreased tail fin development to severely diminished tail development (Furthauer et al., Dev. Biol. 214:181-196, 1999). These classic morphologies resulting from BMP agonism or antagonism provide a method for rapid confirmation of activity in vivo, provided compounds are bioavailable, as described above. This assay is also highly sensitive to non-specific toxicity, which typically results in less stereotyped abnormalities or death.

Zebrafish embryos exposed to 5-20 uM of Compound C/Dorsomorphin display progressive dorsalization, with the classic morphologic findings of that found in noggin overexpressing zebrafish embryos, consistent with specific inhibition of BMP signaling (Figure 9D-F) and in the absence of other nonspecific toxicity. It should be noted that effective concentrations in zebrafish may be distinct from those seen in cultured cells, due to differences in bioavailability of molecules to an intact zebrafish embryo.

BMP ligands are known to induce osteoblast differentiation and osteogenic gene expression in a variety of cell types, including the multipotent C2C12 myofibroblast cell line. This activity is a reasonable surrogate for the bone forming potential of various BMP ligands. We found that Compound C/Dorsomorphin at 4 µM completely inhibits the osteogenic differentiation of C2C12 cells (measured by alkaline phosphatase activity) induced by BMP4 or BMP6 (Figure 10) in vitro. This inhibition is more potent than that seen with pretreatment of cells with 100 ng/ml of noggin, a preferential inhibitor ofBMP2 and BMP4 activity. Moreover, this inhibition of osteogenic differentiation was not associated with cell toxicity (bottom panel), as assayed by cell counts after treatment with BMP, Compound C/Dorsomorphin, noggin, or their various combinations. The results of additional experiments showing the effects of Compound C/Dorsomorphin on C2C12 differentiation are shown in Figure 11.

Compound C/Dorsomorphin effectively neutralizes BMP signaling in wild type cells and in mutant cells that lack the BMP type II receptor. We also find that Compound C/Dorsomorphin effectively inhibits the activity of BMPs 2, 4, 6, 7, and 9 in wild type and BMP type II receptor knockout cells. These attributes, and the known (less potent) activity of Compound C/Dorsomorphin in inhibiting the serine-threonine kinase activity of AMP kinase indicates that Compound C/Dorsomorphin may inhibit the activity of the BMP type I receptor (ALK2, ALK3, or ALK6) serine-threonine kinases. Results of experiments showing that Compound C/Dorsomorphin inhibits active forms of type I receptors ALK2, ALK3, and ALK6 are shown in Figure 12 and 21 h, and the results of experiments showing that Compound C/Dorsomorphin disrupts bone mineralization are shown in Figures 13-16.

Structural analogs of Compound C/Dorsomorphin can also be tested and used in the methods of the invention. Examples of such analogs, which have previously been described as inhibiting VEGFR2 with varying degrees of specificity and potency, are shown in Figure 17.

Additional experiments characterizing Compound C/Dorsomorphin are described below, in Example 2.

### Example 2

Bone morphogenetic protein (BMP) signals coordinate developmental patterning, organogenesis, and tissue remodeling, and play essential physiological roles in mature organisms. Here we describe the first known small molecule inhibitor of BMP signaling, Compound C/Dorsomorphin, identified in a screen for compounds that perturb dorsoventral axis formation in zebrafish. Exposure to Compound C/Dorsomorphin during early embryogenesis recapitulated a full spectrum of dorsalization defects associated with genetic disruption of BMP signaling and also compensated for knockdown of the endogenous BMP antagonist chordin. Compound C/Dorsomorphin was found to selectively inhibit BMP type I receptors ALK2, ALK3, and ALK6 and thus block BMP-mediated SMAD1/5/8 phosphorylation, target gene transcription, and osteogenic differentiation. Using Compound C/Dorsomorphin, we examined the role of BMP signaling in iron homeostasis in zebrafish and mice. In vitro, Compound C/Dorsomorphin inhibited BMP-, hemojuvelin-, and interleukin 6-mediated transcription of the systemic iron regulator hepcidin, suggesting that BMP receptors regulate hepcidin induction by all of these stimuli. *In vivo,* systemic challenge with iron rapidly induced SMAD1/5/8 phosphorylation and hepcidin expression in the liver, while treatment with Compound C/Dorsomorphin blocked SMAD1/5/8 phosphorylation, normalized hepcidin expression, and increased serum iron levels. These findings suggest an essential physiological role for hepatic BMP signaling in iron-hepcidin homeostasis and that pharmacological BMP inhibition might be used to treat anemia of chronic disease caused by elevated hepcidin levels.

### Results

### Compound C/Dorsomorphin induces dorsalization in zebraftsh embryos

As described above in Example 1, small molecule inhibitors of BMP signaling were sought using an *in vivo* screening assay based on the premise that BMP antagonists would dorsalize developing zebrafish embryos. Over 7,500 compounds were tested from our small molecule collection, which consists of known bioactive molecules, FDA-approved drugs, and a commercial molecular diversity library. Zebrafish embryos were arrayed into 96-well plates and incubated with compounds starting 4 hours post fertilization (hpf) and assessed visually at 24 and 48 hpf. One compound, which we call Compound C/Dorsomorphin (Figure 18a), was identified that produces dramatic and reproducible dorsalization of zebrafish embryos, as manifested by the expansion of structures derived from the dorsal pole of spherical embryos at the expense of structures derived from the ventral pole (Nguyen et al., Dev. Biol. 199:93-110, 1998; Furthauer et al., Dev. Biol. 214:181-196, 1999; Mintzer et al., Development 128:859-869, 2001; Schmid et al., Development 127:957-967, 2000) (Figure 18b-e).

The extent of dorsalization induced by Compound C/Dorsomorphin varied as a function of dose and timing. When added at 6-8 hpf, Compound C/Dorsomorphin caused mild dorsalization manifest as the absence of the ventral tail fin (Figure 18b-c) similar to that of noggin-overexpressing and *lost-a-fin* (ALK8 mutant) zebrafish (Mullins et al., Development 123:81-93, 1996; Furthauer et al., Dev. Biol.214:181-196,1999). Zebrafish treated with Compound C/Dorsomorphin at 6 hpf occasionally exhibited an ectopic tail appendage in addition to the absent ventral fin (Figure 18d), resembling transgenic zebrafish expressing a heat-shock-inducible dominant negative BMP type I receptor after the shield stage (Pyati et al., Development 132:2333-2343, 2005). When Compound C/Dorsomorphin was added to embryos at 1-2 hpf, caudal and posterior structures of the tail derived from the embryonic ventral pole were more profoundly disrupted (Figure 18e), resembling fish with more severe BMP signaling defects (Mullins et al., Development 123:81-93, 1996). In fact, the addition of compound at 2 hpf led to altered gastrulation and somitogenesis similar to that observed in *lost-a-fin* fish (Mullins et al., Development 123:81-93, 1996), with ovoid dorsalized morphology at the bud stage (Figure 18f-g), and abnormal tailbud morphology at the 10-somite stage (Figure 18h-i). Treatment of embryos with ≥5 µM Compound C/Dorsomorphin at ≤8 hpf phenocopied with high penetrance the spectrum of dorsalization defects observed in zebrafish with defective BMP signaling (Figure 19 and Tables 1 and 2) but did not result in cyclopia, a phenotype associated with defective TGF-β signaling (Sampath et al., Nature 395:185-189, 1998).

Compound C/Dorsomorphin, as described above, is a molecule previously shown to antagonize AMP-activated kinase (AMPK) activity *in vitro* with an estimated functional Kᵢ of 10 to 20 µM (Zhou et al., J. Clin. Invest. 108:1167-1174,2001). To test whether AMPK inhibition contributes to the effects of Compound C/Dorsomorphin on dorsoventral axis formation, we tested structurally-unrelated inhibitors of AMPK activity such as C75 and Ara-A (Kim et al., J. Biol. Chem. 279:19970-19976, 2004; Yoon et al., Diabetes 55:2562-2570, 2006). These inhibitors did not dorsalize zebrafish embryos, but instead caused non-specific toxicity such as developmental delay and death that was not observed with dorsalizing concentrations of Compound C/Dorsomorphin (Table 3). Moreover, knockdown of AMPK activity in zebrafish by morpholino injection did not cause dorsalization (Figure 20). Compound C/Dorsomorphin is structurally related to a series of compounds that have been shown to inhibit the activity of the receptor tyrosine kinase KDR (Fraley et al., Bioorg. Med. Chem. Lett. 12:2767-2770, 2002). At the doses of Compound C/Dorsomorphin used in our experiments, however, we did not observe any of the known effects of KDR inhibition on zebrafish vascular development, such as disrupted formation of intersomitic blood vessels (Habeck et al., Curr. Biol. 12:1405-1412,2002) (Figure 18c-f). Therefore, Compound C/Dorsomorphin-induced dorsalization is not caused by AMPK or KDR inhibition.

### Compound ClDorsomorphin suppresses ventral marker and expands dorsal marker expression

To confirm the impact of Compound C/Dorsomorphin upon dorsoventral axis formation, the expression of dorsal and ventral markers were examined. Expression of the ventral marker *evel* at the 18-somite stage was reduced in Compound C/Dorsomorphin-treated embryos as compared to controls (Figure 18j-k), consistent with dorsalization (Mullins et al., Development 123:81-93, 1996). At the 6-somite stage, *pax2.1* expression demarcates the optic stalk, mid-hindbrain boundary, and otic vesicles (Little et al., Birth Defects Res. C Embryo Today 78:224-242, 2006). Compound C/Dorsomorphin treatment induced expansion of these dorsal tissues (Figure 181-m). Similarly, Compound C/Dorsomorphin induced lateral expansion of *krox20,* a marker of rhombomeres 3 and 5, and *myoD,* a marker of posterior myotomes (Figure 18n-o). Thus, Compound C/Dorsomorphin induced changes in the expression of dorsal and ventral markers consistent with the effects of dorsalization seen in zebrafish BMP pathway mutants (Little et al., Development 131:5825-5835, 2004).

### Compound ClDorsomorphin reverses ventralization in chordin morphant embryos

Since treatment with Compound C/Dorsomorphin mimics BMP antagonism in zebrafish, it was postulated that Compound C/Dorsomorphin might rescue ventralization caused by loss of the endogenous BMP antagonist *chordin* (Hammerschmidt et al., Development 123:143-151, 1996; Nasevicius et al., Nat. Genet. 26:216-220, 2000). Zebrafish were injected with *chordin* morpholino (1 ng) at the 1 cell stage, causing moderate ventralization with expansion of tail structures and the intermediate cell mass (ICM) (Figure 18p) (Nasevicius et al., Nat. Genet. 26:216-220, 2000; Leung et al., Dev. Biol. 277:235-254,2005). Compound C/Dorsomorphin treatment of *chordin* morphants at 2 hpf prevented ventralization and ICM expansion (Figure 18q). Therefore, Compound C/Dorsomorphin treatment compensates for the loss of an endogenous BMP antagonist in zebrafish embryos, confirming its function as a BMP antagonist.

### Compound C/Dorsomorphin inhibits SMAD-dependent but not SMAD-independent BMP signals

The effect of Compound C/Dorsomorphin upon BMP signaling was tested *in vitro.* Cultured murine pulmonary artery smooth muscle cells (PASMCs) express a complement of BMP receptors and in response to diverse BMP ligands exhibit SMAD1/5/8 activation, MAPK p38 activation, and transcription of inhibitor of differentiation *(Id)* genes, a group of dominant-negative acting basic helix-loop-helix transcription factors which generally act to promote proliferation and inhibit differentiation (Yu et al., J. Biol. Chem. 280:24443-24450, 2005; Miyazono et al., Sci STKE 2002, PE40, 2002). Compound C/Dorsomorphin inhibited BMP4-induced phosphorylation of BR-SMADs in a dose-dependent manner (IC₅₀ = 0.47 µM), without affecting MAPK p38 activation (Figure 21a-b). The unique ability of Compound C/Dorsomorphin to functionally separate SMAD1/5/8 and MAPK p38 activation suggests that BMPs activate these effectors by distinct mechanisms. In contrast to Compound C/Dorsomorphin, noggin inhibited BMP4-induced phosphorylation of both SMAD1/5/8 and MAPK p38 (Figure 21c), consistent with inhibition of SMAD-dependent and SMAD-independent signaling by sequestration of BMP ligands. Compound C/Dorsomorphin (4 µM) inhibited the activity of structurally diverse BMPs, blocking BR-SMAD activation by BMPs 2, 4, 6, and 7 (Figure 21d) and reducing phosphorylated SMAD levels in untreated cells to below basal levels. Compound C/Dorsomorphin did not inhibit SMAD2 activation by TGF-β1 or Activin A at concentrations equal to or greater than those that were inhibitory to BMP signaling (Figure 21 e-f). These results are depicted quantitatively in Figure 22a-e.

### Compound C/Dorsomorphin inhibits BMP type I receptor function

To test whether or not Compound C/Dorsomorphin inhibits the function of BMP type II receptors, we tested Compound C/Dorsomorphin in PASMCs lacking the BMP type II receptor (BMPR-II). BMPR-II-deficient cells utilize the Activin type IIa receptor (ActR-IIa) instead of BMPR-II to activate the type I BMP receptors (Yu et al., J. Biol. Chem. 280:24443-24450, 2005). Compound C/Dorsomorphin inhibited signaling by BMPs 2, 4, 6, and 7 in BMPR-II-deficient cells (Figure 23) demonstrating that Compound C/Dorsomorphin inhibits BMP signals in the presence or absence of BMPR-II. This finding suggested Compound C/Dorsomorphin might inhibit signaling of BMP type I receptors downstream of type II receptors. To test this hypothesis, cells were transfected with constitutively-active (ca) forms of the BMP type I receptors ALK2, ALK3, ALK6. Transfection with caALK2, caALK3, or caALK6 increased the phosphorylation of SMAD1/5/8 in cell extracts, while preincubation with Compound C/Dorsomorphin (10 µM) inhibited this increase in phosphorylated SMAD1/5/8 (Figure 21 g). Similarly, transfection with caALK2, caALK3, or caALK6 increased *Idl* promoter (BRE-Luc) activity by 5- to 12-fold in the absence of ligand. Compound C/Dorsomorphin inhibited the activity of caALK2, caALK3, and caALK6, with apparent selectivity for caALK2 and caALK3 over caALK6 (Figure 21 h). At similar concentrations, Compound C/Dorsomorphin did not inhibit the function of constitutively-active ALK4, ALK5, or ALK7, the TGF-β and Activin type I receptors (Figure 24). The ability of Compound C/Dorsomorphin to block BMP-mediated SMAD1/5/8 phosphorylation and to inhibit the ability of activated BMP type I receptors to phosphorylate SMAD1/5/8 and induce downstream transcription argues strongly that Compound C/Dorsomorphin inhibits BMP type I receptor kinase function.

### Compound C/Dorsomorphin inhibits BMP-mediated osteogenic differentiation and bone mineralization

The ability of Compound C/Dorsomorphin to block BMP-mediated signaling and transcriptional activity suggested that Compound C/Dorsomorphin might block BMP-induced osteoblast differentiation. Alkaline phosphatase activity in myofibroblast C2C12 cells was efficiently induced by BMP4 and to a lesser extent by BMP6 after five days of culture (Figure 25a). This activity was almost completely abolished by the addition of Compound C/Dorsomorphin at 4 µM, a concentration at which no significant effects upon cell count (Figure 25b) were observed. These findings suggest that, consistent with inhibition of BMP function, Compound C/Dorsomorphin inhibits BMP-induced osteogenic differentiation in multipotent mesenchyme-derived cells without cytotoxicity.

To test whether or not the effects of Compound C/Dorsomorphin on osteogenic differentiation *in vitro* could be extended to osteogenesis *in vivo,* we analyzed bone mineralization in zebrafish embryos. To circumvent dorsalization during early embryogenesis, zebrafish were exposed to Compound C/Dorsomorphin after establishment of the dorso ventral axis beginning 24 hpf. Ten days post fertilization, calcein staining was performed to assess bone mineralization. Treatment with Compound C/Dorsomorphin resulted in inhibition of bone mineralization, with fewer calcein-stained vertebral segments when compared to control larvae (Figure 25c-f). Despite altered bone mineralization, fish treated with Compound C/Dorsomorphin otherwise appeared morphologically and functionally normal by brightfield microscopy (Figure 26).

### Compound C/Dorsomorphin inhibits BMP-, HJV-, and IL-6-induced hepcidin transcription

BMPs, HJV, and IL-6 induce hepcidin expression in cultured hepatocytes (Babitt et al., Nat. Genet. 38:531-539, 2006; Truksa et al., Proc. Natl. Acad. Sci. U.S.A. 103:10289-10293, 2006; Nemeth et al., J. Clin. Invest. 113:1271-1276, 2004; Lee et al., Proc. Natl. Acad. Sci. U.S.A. 101:9263-9265, 2004) and cultured hepatoma cells (Truksa et al., Proc. Natl. Acad. Sci. U.S.A. 103:10289-10293, 2006; Babitt et al., J. Clin. Invest. 117:1933-1939, 2007). HJV functions as a BMP-co-receptor, but it is unclear whether or not IL-6-induced hepcidin expression requires BMP signaling (Wang et al., Cell Metab. 2:399-409, 2005). Treatment with BMP2 (Figure 27a) or transfection with cDNA encoding HJV (Figure 27b) induced transcriptional activity of the hepcidin promoter in hepatoma-derived Hep3B cells. BMP2- and HJV-induced hepcidin promoter activities were both effectively abrogated by the addition of Compound C/Dorsomorphin. HepG2 cells exhibit higher basal hepcidin expression than Hep3B cells (Babitt et al., Nat. Genet. 38:531-539, 2006). Treatment with Compound C/Dorsomorphin reduced basal hepcidin expression by over 50-fold in HepG2 cells and abolished the induction of hepcidin expression by BMP2 (Figure 27c). In contrast to Compound C/Dorsomorphin, treatment of Hep3B cells with Ara-A at concentrations sufficient to inhibit AMPK (Chen et al., Dev. Biol. 291:227-238, 2006) did not impair BMP-induced hepcidin mRNA expression, making it unlikely that Compound C/Dorsomorphin inhibited hepcidin expression via effects on AMPK (Figure 28). Treatment with IL-6 induced hepcidin and Id1 expression in Hep3B cells, while the addition of Compound C/Dorsomorphin blocked IL-6-mediated induction of both hepcidin and Id1 (Figure 21d-e). These findings suggest that BMP-, HJV-, and IL-6-mediated hepcidin expression are all regulated by signaling via BMP type I receptors.

### Compound C/Dorsomorphin inhibits iron-mediated BR-SMAD activation in the liver

*In vivo*, iron induces the hepatic expression of hepcidin to adaptively curtail excess ferroportin-mediated iron transport (Fraenkel et al., J. Clin. Invest. 115:1532-1541, 2005; Nemeth et al., Science 306:2090-2093, 2004), but it was unclear whether or not BMP signaling is involved in this feedback process. To ascertain the potential role of BMP signaling in iron feedback regulation of hepcidin, the effect of systemic iron challenge on hepatic SMAD1/5/8 activation was examined. Intraperitoneal injection of iron-dextran in adult zebrafish led to a nearly 3-fold increase in SMAD1/5/8 phosphorylation in liver extracts within 1 hour, as compared to dextran-injected controls (Figure 29a). When zebrafish were co-injected with iron-dextran and dorsomorphin, SMAD1/5/8 phosphorylation decreased by nearly 3-fold as compared to fish injected with iron-dextran and vehicle (Figure 29b). Similarly in mice, intravenous injection of iron-dextran and vehicle led to a more than 3-fold increase in SMAD1/5/8 phosphorylation in liver extracts after 1 hour, as compared to mice injected with dextran and vehicle (Figure 29c). Co-injection of Compound C/Dorsomorphin in mice abolished the iron-mediated increase in hepatic SMAD1/5/8 phosphorylation. These observations in two species demonstrate that hepatic SMAD1/5/8 activation is rapidly induced by iron and is effectively inhibited by Compound C/Dorsomorphin.

### Compound C/Dorsomorphin inhibits iron-induced hepcidin transcription in zebrafish

Although systemic iron challenge is known to induce hepcidin expression *in vivo,* the responsible mechanisms are incompletely understood since the hepcidin promoter lacks recognizable transcriptional elements that are known to respond to changes in iron levels. The observation that iron challenge causes hepatic SMAD1/5/8 activation led to the hypothesis that BMP signaling mediates hepcidin expression in response to iron. Adult zebrafish normally express relatively low levels of hepcidin. After 3 hours, fish treated with iron-dextran and vehicle exhibited a 4-fold increase in hepatic hepcidin mRNA levels compared to fish injected with dextran and vehicle (Figure 29d). Co-treatment of fish with Compound C/Dorsomorphin inhibited the increase of hepcidin expression seen with iron-dextran injection, suggesting that iron induces hepcidin expression via a BMP-dependent mechanism.

### Compound C/Dorsomorphin decreases basal hepatic hepcidin expression and induces hyperferremia in mice

Wild-type C57BL/6 adult mice fed a standard iron-replete diet express substantial hepcidin (Nicolas et al., Proc. Natl. Acad. Sci. U.S.A. 99:4596-4601, 2002) presumably induced by abundant provision of iron. The ability of Compound C/Dorsomorphin to block iron-induced hepcidin expression in zebrafish led to the hypothesis that Compound C/Dorsomorphin could inhibit basal hepcidin expression and increase serum iron levels in iron-replete mice. Six hours after Compound C/Dorsomorphin was administered intravenously, hepatic hepcidin mRNA levels were reduced to one-third that of vehicle-injected mice (*P*<0.01) (Figure 29e). Alterations in hepcidin levels affect serum iron concentrations within 24 hours via the altered mobilization of intracellular iron by ferroportin (Nemeth et al., J. Clin. Invest. 113:1271-1276, 2004). Administration of Compound C/Dorsomorphin over 24 hours led to a 60% increase in total serum iron concentrations (Figure 29f). Compound C/Dorsomorphin treatment is therefore effective in reducing basal levels of hepcidin expression and increasing serum iron concentrations in adult mice.

### Methods

### Screening for small molecules that perturb dorso-ventral axis in zebrafish embryos

Small molecule screening in zebrafish was performed as previously described (Peterson et al., Nat. Biotechnol. 22:595-599, 2004) with several modifications. Pairs of wild-type zebrafish were mated, and newly-fertilized eggs were arrayed in triplicate in 96-well plates containing E3 buffer. At 4 hours post fertilization (hpf), compounds were added to wells at 5-10 µM. Embryos were incubated at 28.5°C, and gross morphology of embryos was examined at 12, 24, and 48 hpf for dorsalization or ventralization of embryonic axis, as previously described (Mullins et al., Development 123:81-93, 1996). In total, 7,570 compounds were screened, including synthetic screening compounds (Chembridge Corporation, 5,580 small molecules) and known bioactive compounds (Microsource Discovery Systems, 1,840 small molecules and Sigma-Aldrich, 150 small molecules). For follow up studies, Compound C/Dorsomorphin and Ara-A (adenine 9-β-D-arabinofurano-side) were purchased from Sigma (St. Louis, MO), and C75 was purchased from EMD Biosciences (San Diego, CA).

### Wholemount zebrafish in situ hybridization

In situ hybridization was performed as previously described (Westerfield, The Zebrafish Book, (University of Oregon Press, Eugene, OR, 1993)). Zebrafish krox20, pax2.1, and MyoD probes were produced as previously described (Weinberg et al., Development 122:271-280, 1996).

### Cell culture

Wild-type and BMPR-II-deficient PASMC were isolated as previously described (Yu et al., J. Biol. Chem. 280:24443-24450, 2005), and cultured in RPMI medium (Invitrogen) supplemented with 10% FBS. C2C12 cells (American Type Culture Collection, Manassas, VA) were cultured in DMEM supplemented with glutamine and 10% FBS. HepG2 cells and Hep3B cells (ATCC) were cultured in Minimal Essential Alpha Medium with L-glutamine (α-MEM, Invitrogen) containing 10% FBS. For studies of SMAD activation *in vitro,* PASMC were incubated in 0.5% FCS RPMI for 24 hours, followed by preincubation with drug compounds, noggin, or vehicle for 30 minutes, followed the addition of recombinant BMPs 2, 4, 6, or 7, or TGF-β1 or Activin A (R&D Systems, Minneapolis, MN) for 30 minutes.

### Immunoblot analysis of BMP and TGF-β responsive SMAD phosphorylation

Cell extracts or tissues were mechanically homogenized in SDS-lysis buffer (62.5 mM Tris-HCl (pH 6.8), 2% SDS, 10% glycerol, 50 mM DTT, 0.01 % bromophenol blue); separated by SDS-PAGE; immunoblotted with anti-phospho-SMAD1/5/8, anti-phospho-SMAD2 (Cell Signaling, Beverly, MA), or anti-α-tubulin (Upstate/Millipore, Billerica, MA) Abs; and visualized using ECL Plus (GE Healthcare, Piscataway, NJ). Levels of immunoreactive proteins were quantitated by ImageQuant software on a Storm phosphorimager (GE Healthcare).

### Alkaline phosphatase activity

C2C12 cells were seeded into 96 well plates at 2000 cells per well in DMEM supplemented with 2% FBS. Wells were treated in quadruplicate with BMP ligands and Compound C/Dorsomorphin or vehicle. Cells were harvested after 5 days in culture with 50 µl Tris buffered saline/1% Triton X-100. Lysates were added to p-nitro-phenylphosphate reagent in 96-well plates (Sigma) for 1 hour, and alkaline phosphatase activity expressed as absorbance at 405 nM. Cell viability and quantity were measured by Cell-titer Glo (Promega) and binding of nuclear dye CyQuant (Invitrogen), respectively, using replicate wells treated identically to those used for alkaline phosphatase measurements.

### Zebrafish bone mineralization

Wildtype zebrafish embryos were raised in E3 buffer containing phenylthiourea (PTU) (Westerfield, The Zebrafish Book, (University of Oregon Press, Eugene, OR, 1993)). At 1 day post fertilization (dpf), embryos were treated with Compound C/Dorsomorphin (1-4 µM) or DMSO vehicle. At 5 dpf and onward, larvae were fed for 1 hour every other day. Following each feeding, residual food was washed out, and media replaced with E3 containing Compound C/Dorsomorphin or vehicle. At 10 dpf, larvae were immersed in calcein (0.2%) for 30 minutes, as previously described (Du et al., Dev. Biol. 238:239-246, 2001). Embryos were washed repeatedly in E3 buffer for 3 hours to remove unbound calcein and anesthestized with tricaine. Calcified skeletal structures were visualized by green fluorescence and the number of vertebral bodies counted.

### BMP responsive element and hepcidin promoter luciferase reporter assays

Murine PASMCs grown to 50% confluence in 6-well plates were transiently transfected with 0.3 µg *IdI* promoter luciferase reporter construct (BRE-Luc, Dr. Peter ten Dijke, Leiden University Medical Center, Netherlands (Korchynskyi et al., J. Biol. Chem. 277:4883-4891, 2002)) in combination with 0.6 µg plasmids expressing constitutively-active forms of BMP type I receptors (caALK2, caALK3, or caALK6, kindly provided by Dr. Kohei Miyazono, University of Tokyo, Japan (Fujii et al., Mol. Biol. Cell 10:3801-3813, 1999)), using Fugene6 (Roche). To assess Activin and TGF-β type I receptor function, PASMCs were transiently transfected with 0.3 µg *PAI-1* promoter luciferase reporter construct (CAGA-Luc, Dr. Peter ten Dijke (Dennleret al., Embo J. 17:3091-3100, 1998)) in combination with 0.6 µg plasmids expressing constitutively active forms of type I receptors (caALK4, caALK5, and caALK7, also provided by Dr. Kohei Miyazono (Okadome et al., J. Biol. Chem. 271:21687-21690, 1996; Shimizu et al., Genes Cells 3:125-134, 1998)). For both reporters, 0.2 µg pRL-TK Renilla luciferase (Promega) was used to control for transfection efficiency. PASMCs were incubated with Compound C/Dorsomorphin (4-10 µM) or vehicle starting one hour after transfection, for 24 hours or completion of the experiment. Cell extracts were harvested, and relative promoter activity quantitated by the ratio of firefly to Renilla luciferase activity using the dual luciferase assay kit (Promega). HepG2 or Hep3B cells were transiently transfected with 2.5 µg hepcidin promoter luciferase reporter (Babitt et al., Nat. Genet. 38:531-539, 2006) in combination with 0.25 µg pRL-TK to control for transfection efficiency, with or without 20 ng cDNA encoding FLAG-tagged human HJV. Two days after transfection, HepG2 and Hep3B cells were incubated in 1% FBS α-MEM for 6 hours, treated with Compound C/Dorsomorphin (10 µM) or vehicle for 30 minutes, and then incubated for 16 hours in the presence or absence of BMP2 (25 ng/ml). Hepcidin promoter activity was measured by luciferase assay as above.

### Iron-dextran injections

Adult fish were anesthetized with tricaine and injected with 10 µl of iron-dextran solution (100 mg/ml, average dextran MW = 5000, Sigma) into the abdominal cavity with Compound C/Dorsomorphin (23 µg/g) or vehicle (DMSO). Control fish were injected with 10 µl of dextran (average MW=5000, Sigma). Fish were revived in water. One hour after injection, fish were anesthetized on ice, and livers were collected into 200 µL SDS-lysis buffer and homogenized mechanically. Fifteen µL of protein extract was fractionated by SDS-PAGE and immunoblotted, as described above. Three hours after injection, total RNA was extracted from mechanically homogenized zebrafish livers using Trizol reagent (Invitrogen).

Twelve-week old C57BL/6 mice raised on a standard diet (Isopro RMH 3000, Prolab) were injected via the tail vein with 0.2 g/kg of dextran (average MW=5000, Sigma) or 0.2 g/kg of iron-dextran USP (DexFerrum, American Regent Laboratories). Dextran was injected with vehicle only, whereas iron-dextran was injected with either vehicle or Compound C/Dorsomorphin (10 mg/kg). One hour after injection, mice were sacrificed, and liver segments were collected in 500 µl SDS-lysis buffer and mechanically homogenized. Twenty µL of liver extracts were resolved by SDS-PAGE and immunoblotted. Total RNA was harvested using Trizol from mechanically-homogenized mouse livers (6 hours after injection with a single intraperitoneal dose of Compound C/Dorsomorphin (10 mg/kg) or DMSO).

### Quantitative hepcidin RT-PCR

Hep3B or HepG2 cells were incubated in 1% FCS α-MEM for 6 hours in the presence or absence of Compound C/Dorsomorphin (10 µM) with or without BMP2 (25 ng/ml) or human IL-6 (R&D Biosystems, 100 ng/ml), and RNA then extracted from cultured cells with Trizol. Quantitative RT-PCR was performed for mRNA encoding Id1 or human hepcidin (*HAMP*) as previously described (Babitt et al., Nat. Genet. 38:531-539, 2006; Yu et al., J. Biol. Chem. 280:24443-24450, 2005), and normalized to 18S ribosomal RNA (rRNA) or or β-actin mRNA levels.

In murine and zebrafish experiments, quantitative RT-PCR was performed on cDNA generated from equal quantities of RNA extracted from livers of treated animals. Triplicate or quadruplicate experiments were performed as indicated. Expression levels of mRNA encoding zebrafish hepcidin and murine hepcidin (*HAMP1*) were measured as previously described (Fraenkel et al., J. Clin. Invest. 115:1532-1541, 2005; Wang et al., Cell Metab. 2:399-409, 2005), and normalized to liver fatty acid binding protein mRNA (zebrafish) or 18S rRNA (murine) levels respectively.

### Serum iron measurements

Whole blood was collected from mice by cardiac puncture into Microtainer serum separator tubes (BD Scientific), and serum was isolated according to the manufacturer's instructions. Serum iron levels were measured by colorimetric assay using the Iron/UIBC kit (Thermo Fisher Scientific, Waltham, MA).

### Statistical analysis

The statistical significance of compared measurements was measured using the student's two-tailed T-test or one-way ANOVA with Bonferroni correction. Hill plot analysis of Compound C/Dorsomorphin inhibition of SMAD1/5/8 phosphorylation was performed using GraFit software (Erithacus Software, Surrey, England).

### Other Embodiments

All publications and patents cited in this specification are herein incorporated by reference as if each individual publication or patent were specifically and individually indicated to be incorporated by reference. Use of singular forms herein, such as "a" and "the," does not exclude indication of the corresponding plural form, unless the context indicates to the contrary. Although the invention has been described in some detail by way of illustration and example for purposes of clarity of understanding, it will be readily apparent to those of ordinary skill in the art in light of the teachings of the invention that certain changes and modifications may be made thereto without departing from the spirit or scope of the appended claims.

Other embodiments are within the following items.
1. A method of identifying a compound that inhibits Bone Morphogenetic Protein (BMP) or Transforming Growth Factor-β (TGF- β) signaling, the method comprising contacting a non-human embryo with a candidate compound and observing the phenotype of the embryo, wherein:
   detection of dorsalization of the embryo, relative to an untreated control, indicates the identification of a compound that inhibits BMP signaling; and
   detection of ventralization of the embryo, relative to an untreated control, indicates the identification of a compound that enhances or initiates BMP signaling; and
   detection of cyclopia or reduction in head size, relative to an untreated control, indicates the identification of a compound that inhibits TGF-β signaling.
2. The method of item 1, wherein the non-human embryo is selected from the group consisting of zebrafish, xenopus, medaka, and Drosophila embryos.
3. A method of identifying a compound that modulates Bone Morphogenetic Protein (BMP) or Transforming Growth Factor-β (TGF-β) signaling, the method comprising:
   contacting cells with a candidate compound;
   permeabilizing the plasma and nuclear membranes of the cells and precipitating proteins in the cells by methanol treatment;
   cross-linking precipitated proteins with glutaraldehyde;
   quenching the glutaraldehyde from the cells with a bifunctional amine; and
   determining the effect of the candidate compound on phosphorylation of SMAD2 or SMAD1/5/8 in compound treated cells, relative to untreated cells, wherein detection of increased phosphorylation of SMAD1/5/8 indicates the identification of a compound that activates BMP signaling, detection of decreased phosphorylation of SMAD1/5/8 indicates the identification of a compound that inhibits BMP signaling, detection of increased phosphorylation of SMAD2 indicates the identification of a compound that activates TGF-β signaling, and detection of decreased phosphorylation of SMAD2 indicates the identification of a compound that inhibits TGF-β signaling.
4. The method of item 3, wherein the methanol is ice-cold and the methanol treatment is carried out for 10-15 minutes.
5. The method of item 3, wherein glutaraldehyde solution comprises a concentration of glutaraldehyde of 0.25% to 2%, and the glutaraldehyde treatment is carried out for 10 to 15 minutes.
6. The method of item 3, wherein the bifunctional amine is selected from the group consisting of lysine, ethylenediamine, and a phenylenediamine, which are used at a concentration of 25-200 mM, and at a pH of 8-10.
7. The method of item 3, wherein detection of phosphorylation of SMAD1/5/8 or SMAD2 is carried out by use of an Enzyme Linked Immunosorbent Assay (ELISA).
8. A method of treating or preventing a disease or condition in a subject that would benefit by inhibition of Bone Morphogenetic Protein (BMP) signaling, the method comprising administering to the subject a compound that selectively inhibits BMP signaling, relative to Transforming Growth Factor-β (TGF-β) signaling.
9. The method of item 8, wherein the compound is 6-[4-(2-piperidin-1-yl-ethoxy)-phenyl)]-3-pyridin-4-yl-pyrrazolo[1,5-a]-pyrimidine (Compound C/Dorsomorphin).
10. The method of item 8, wherein the compound is selected from those shown in Figure 17.
11. The method of item 8, wherein the disease or condition is selected from the group consisting of familial or sporadic primary pulmonary hypertension, hereditary hemorrhagic telangectasia syndrome, cardiac valvular malformations, cancer (in modulating the growth of primary tumors or metastases, as a primary therapy or providing an adjuvant therapy for traditional cancer therapies), anemia, vascular calcification, atherosclerosis, valve calcification, renal osteodystrophy,
   inflammatory disorders, infections with viruses, bacteria, fungi, tuberculosis, and parasites, and Fibrodysplasia Ossificans Progressiva.
12. The method of item 11, wherein the cancer is selected from the group consisting of breast carcinoma, prostate carcinoma, renal cell carcinoma, bone metastasis, osteosarcoma, and multiple myeloma.
13. The method of item 11, wherein the inflammatory disorder is ankylosing spondylitis.
14. A method of treating or preventing a disease or condition in a subject that would benefit by inhibition of Transforming Growth Factor-β (TGF-β) signaling, the method comprising administering to the subject a compound that selectively inhibits TGF-β signaling, relative to Bone Morphogenetic Protein (BMP) signaling.
15. The method of item 14, wherein the disease or condition is a fibrotic disease or a valvular or aortic abnormality of Marfan's syndrome.
16. The method of item 15, wherein the fibrotic disease is of the liver, kidneys, or lungs.
17. A pharmaceutical composition comprising Bone Morphogenetic Protein (BMP) inhibitor.
18. The pharmaceutical composition of item 17, wherein the Bone Morphogenetic Protein (BMP) inhibitor is 6-[4-(2-piperidin-1-yl-ethoxy)-phenyl)]-3-pyridin-4-yl-pyrrazolo[1,5-a]-pyrimidine (Compound C/Dorsomorphin).
19. A method of modifying the phenotype of a cell, the method comprising contacting the cell with a Bone Morphogenetic Protein (BMP) inhibitor.
20. The method of item 19, wherein the cell is an embryonic stem cell or an adult stem cell.
21. The method of item 20, wherein the Bone Morphogenetic Protein (BMP) inhibitor allows for the expansion or differentiation of said stem cells ex vivo or in vivo for therapeutic or diagnostic purposes.
22. The method of item 19, wherein the BMP inhibitor is 6-[4-(2-piperidin-1-yl-ethoxy)-phenyl)]-3-pyridin-4-yl-pyrrazolo[1,5-a]-pyrimidine (Compound C/Dorsomorphin).

## Claims

1. A small molecule that selectively inhibits BMP signaling, relative to Transforming Growth Factor-β (TGF-β) signaling, for use for treating or preventing a disease or condition in a subject that would benefit from inhibition of Bone Morphogenetic Protein (BMP) signaling.

2. The small molecule of claim 1, wherein the disease or condition is selected from the group consisting of familial or sporadic primary pulmonary hypertension, hereditary hemorrhagic telangectasia syndrome, cardiac valvular malformations, cancer (in modulating the growth of primary tumors or metastases, as a primary therapy or providing an adjuvant therapy for traditional cancer therapies), anemia, vascular calcification, atherosclerosis, valve calcification, renal osteodystrophy, inflammatory disorders, infections with viruses, bacteria, fungi, tuberculosis, and parasites, and Fibrodysplasia Ossificans Progressiva.

3. The small molecule of claim 2, wherein the cancer is selected from the group consisting of breast carcinoma, prostate carcinoma, renal cell carcinoma, bone metastasis, osteosarcoma, and multiple myeloma.

4. The small molecule of claim 2, wherein the inflammatory disorder is ankylosing spondylitis.

5. The small molecule of any preceding claim, wherein the small molecule is 6-[4-(2-piperidin-1-yl-ethoxy)-phenyl)]-3-pyridin-4-yl-pyrrazolo[1,5-a]-pyrimidine (Compound C/Dorsomorphin).

6. The small molecule of any of claims 1-4, wherein the small molecule is selected from those shown in Figure 17.

7. A pharmaceutical composition comprising a Bone Morphogenetic Protein (BMP) inhibitor that is a small molecule.

8. The pharmaceutical composition of claim 7, wherein the small molecule is 6-[4-(2-piperidin-1-yl-ethoxy)-phenyl)]-3-pyridin-4-yl-pyrrazolo[1,5-a]-pyrimidine (Compound C/Dorsomorphin).

9. The pharmaceutical composition of claim 7, wherein the small molecule is selected from those shown in Figure 17.

10. The pharmaceutical composition of any of claims 7-9, wherein the pharmaceutical composition is for oral administration.
